(19) Europäisches Patentamt / European Patent Office / Office européen des brevets

(11) **EP 3 976 636 B1**

(12) **EUROPEAN PATENT SPECIFICATION**

(45) Date of publication and mention of the grant of the patent:
**21.05.2025 Bulletin 2025/21**

(21) Application number: **20727322.8**

(22) Date of filing: **27.05.2020**

(51) International Patent Classification (IPC):
**C07K 14/47** (2006.01)

(52) Cooperative Patent Classification (CPC):
**C07K 14/47**

(86) International application number:
**PCT/EP2020/064651**

(87) International publication number:
**WO 2020/239809 (03.12.2020 Gazette 2020/49)**

(54) **METHOD FOR PREPARING IRON-DEPLETED FERRITIN AND USE THEREOF**

VERFAHREN ZUR HERSTELLUNG VON EISENARMEM FERRITIN UND DESSEN VERWENDUNG

PROCÉDÉ DE PRÉPARATION DE FERRITINE APPAUVRIE EN FER ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.05.2019 EP 19177012**

(43) Date of publication of application:
**06.04.2022 Bulletin 2022/14**

(73) Proprietor: **PreviPharma Consulting GmbH**
**68167 Mannheim (DE)**

(72) Inventors:
• **KIESSIG, Stephan T.**
**69168 Wiesloch (DE)**
• **MANDAGO, Maurice**
**69250 Schönau (DE)**

(74) Representative: **Jacobi, Markus Alexander**
**Patentanwälte**
**Isenbruck Bösl Hörschler PartG mbB**
**Eastsite One**
**Seckenheimer Landstrasse 4**
**68163 Mannheim (DE)**

(56) References cited:
JP-A- 2004 217 984    US-A- 5 358 722
US-A1- 2011 287 033    US-A1- 2012 195 976

• MOGLIA ITALO ET AL: "An optimized low-cost protocol for standardized production of iron-free apoferritin nanocages with high protein recovery and suitable conformation for nanotechnological applications", JOURNAL OF INORGANIC BIOCHEMISTRY, ELSEVIER INC, US, vol. 183, 21 November 2017 (2017-11-21), pages 184 - 190, XP085383528, ISSN: 0162-0134, DOI: 10.1016/J.JINORGBIO.2017.11.019
• GARRINGER ET AL.: "Effect of Systemic Iron Overload and a Chelation Therapy in a Mouse Model of the Neurodegenerative Disease Hereditary Ferritinopathy", PLOSONE, vol. 11, no. 8, 2016, pages e0161341, XP002800990

**EP 3 976 636 B1**

## Description

The present invention relates to a method for preparing iron-depleted ferritin, in particular apoferritin, wherein said method comprises the provision of an iron ion-adsorbing zeolite, separated from a ferritin-containing fluid by a semipermeable layer. The iron-depleted ferritin, in particular apoferritin, may be used in a method of treating an acute or chronic inflammation and treating and preventing a neurodegenerative disease.

Ferritin is a well-known protein which is involved in the iron metabolism. Ferritin is a universal intracellular protein that stores iron and releases it in a controlled fashion. The protein is produced by almost all living organisms, including algae, bacteria, higher plants, and animals. In humans, it acts as a buffer against iron deficiency and iron overload. Ferritin is found in most tissues as a cytosolic protein, but small amounts are secreted into the serum where it functions as an iron carrier. Plasma ferritin is also an indirect marker of the total amount of iron stored in the body, hence serum ferritin is used as a diagnostic test for iron-deficiency anemia (Wang et al., "Serum ferritin: Past, present and future", Biochimica et Biophysica Acta. 1800 (8):760-769). Iron-containing ferritin can be used to treat anemia.

Ferritin is a globular protein complex consisting of 24 protein subunits forming a nanocage with multiple metal-protein interactions (Theil, "Ferritin protein nanocages - the story", Nanotechnology Perceptions, 2012, 8(1):7-16). It is the primary intracellular iron-storage protein in both prokaryotes and eukaryotes, keeping iron in a soluble and non-toxic form. Ferritin that is essentially free of iron ions and preferably essentially free of other inorganic ions, is called apoferritin. Ferritin is composed of two types of sub-units, H and L. Ferritin H has a potent ferroxidase activity that catalyses the oxidation of iron. Ferritin L plays a role in iron nucleation and protein stability. Ferritin transports iron the bone marrow for the synthesis of haemoglobin and its incorporation into the erythrocytes. Therefore, ferritin is of some interest in the detection and may be even treatment of anaemias, caused by different reasons.

In the human body, in an adult, iron is typically maintained within a range of 4 to 5g by a strict control of its absorption, mobilization, storage, and recycling. Iron excretion is not actively controlled, and skin desquamation is the major mechanism described so far, often accounting for about 1 to 2 mg per day. 90% of iron is often recycled in the human body (e.g. at the degradation of red blood cells), often only 10% is supplemented from the nutrition. Often, 70% of total body iron is bound in heme compounds, about 29% is stored as ferritin and hemosiderin, often <1% is incorporated in heme-containing-enzymes, and often less than 0.2% of iron circulate in the plasma, while being bound to transferrin. 25-30 mg of iron are daily used for the generation of haemoglobin.

Because of its ability to participate directly as a donor or acceptor in electron transfer reactions, iron may become toxic by the generation of highly reactive free radicals that cause lipid peroxidation, DNA strand breaks, and protein modifications that may result in cell death (Harrison PM, Arosio P. The ferritins: molecular properties, iron storage function and cellular regulation. Biochim. Biophys. Acta 1996;1275:161-203. [PubMed: 8695634]).

Ferritin provides a source of metabolic active iron and serves as a radical cytoprotective protein, storing iron that is not consumed for immediate metabolic use. It was shown that the induction of ferritin protects cells from oxidative stress (Regan RF et al.: Ferritin induction protects cortical astrocytes from heme-mediated oxidative injury. Neuroscience. 2002;113(4):985, PMID: 12182902, Shackelford RE et al.: Pharmacological manipulation of ataxia-telangiectasia kinase activity as a treatment for Parkinson's disease. Medical Hypotheses (2005) 64, 736-741). For this reason, ferritin is of some interest in neurodegenerative diseases, like Parkinson's disease. Iron is often associated with oxidative stress that may be associated with neurodegeneration.

This mechanism of neuronal death is proposed as a cause of Parkinson's disease (PD). Although most of researchers agree with this, controversies remain regarding the amounts of iron involved in this process. According to nondestructive methods of assessment of the concentration of the total iron in substantia nigra (SN), there is no difference between Parkinson's disease and control.

However, there is no need for an increase of the total iron in parkinsonian SN to trigger the oxidative stress but only of the non-ferritin-bound labile iron.

Recent studies suggest an increase of this iron in Parkinson's disease (Friedman A et al.: Iron as a cause of Parkinson's disease - a myth or a well-established hypothesis? Parkinsonism Relat Disord. 2009 Dec;15 Suppl 3:S.212-4. doi: 10.1016/S1353-8020(09)70817-X; Mostile G. Iron and Parkinson's disease: A systematic review and meta-analysis. Mol Med Rep. 2017 May;15(5):3383-3389. doi: 10.3892/mmr.2017.6386). This might play a role in other inflammation sites as well. The successful removal of iron at those sites of a chronic inflammation might reduce the level of inflammation, therefore the oxidative stress in the tissue and therefor the tissue damage. This can be of interest due to the fact, that the current therapy for iron overload or oxidative stress is using iron chelators (Garringer et al., "Effect of Systemic Iron Overload and a Chelation Therapy in a Mouse Model of the Neurodegenerative Disease Hereditary Ferritinopathy", PlosOne, 2016, 11(8):e0161341. doi:10.1371/journal.pone.0161341).

A low-cost protocol for standardized production of iron-free apoferritin with high protein recovery and suitable conformation for nanotechnology applications is reported by Moglia et al. in Journal of Inorganic Biochemistry, 2018, 188:184-190.

US 5,358,722 discloses ferritin analogs comprising an apoferritin protein shell and a core substantially devoid of ferrihydrite, e.g. of inorganic composition such as aluminum hydroxide or organic composition such as acetaminophen.

JP-A 2004-217984 relates to a separating agent, which continuously and selectively or non-selectively separates metal ions from an aqueous solution containing various metal ions, and a method for treating an aqueous solution containing metal ions.

Reduction of iron ions (also designatable as ferric ions) such as in a body fluid, such as blood or blood plasma or cerebrospinal fluid, is often achieved by means of addition of chelating agents. Using higher concentrations of chelators for such purpose is, however, bears several drawbacks. It is laborious and often challenging to remove chelators, which are typically soluble agents from a fluid. Further, chelators are often toxic and thus undesired in body fluids. They have despite of the low efficacy some unwanted side effects (Parvu et al., "Ferritin level changes and erythroid improvement in a group of adult polytransfused patients treated with Deferasirox". Clujul Med., 2018, Jul;91(3):288-292).

Therefore, there is a need for providing further means for reducing levels of inorganic ions, in particular iron levels in fluids.

Surprisingly, it was found that iron-depleted ferritin can be used for this purpose and can further be used as a therapeutic agent for treating inflammations and treating and preventing neurogenerative diseases.

It is, however, challenging to prepare sufficiently pure iron-depleted ferritin. As mentioned before, using chelators for this purpose bears several drawbacks. Other means known in the art, such as using porous materials, such as zeolites, have other drawbacks. Such porous materials can remove Fe, Al, Zn, Cd, Cu, Be, Pu, Cs, and other inorganic ions from liquid solutions, during and after the plasma fractionation process and can be easily and efficiently removed from the liquid. However, such materials having a rough surface, such as zeolites, increase coagulation via the intrinsic surface activation pathway. Accordingly, such materials having rough surfaces, such as zeolites, can be used as coagulation-promoting agents (Alam et al.: Hemorrhage Control in the Battlefield: Role of New Hemostatic Agents, Military Medicine, 2005, 170:63-69; Li et al.: Zeolite-based hemostat QuikClot releases calcium into blood and promotes blood coagulation in vitro, Acta Pharmacologica Sinica, 2013, 34:367-372). When, however, coagulation is not desired such as, e.g., in a stored blood or blood plasma sample, the usability of zeolites is hampered due to its tendency to promote undesired coagulation.

Therefore, there is a need for providing further means for preparing iron-depleted ferritin.

Surprisingly, it has been found that iron-depleted ferritin can be obtained from ferritin when using an ion-adsorbing zeolite separated from a ferritin-containing fluid by means of a semipermeable layer that allows the ions to pass through but not polypeptides.

Accordingly, an aspect of the present invention relates to a method for decreasing iron-content of ferritin, wherein said method comprises the following steps:

(i) providing:

(A) a ferritin-containing fluid containing one or more reducing agents (suitable for) reducing $Fe^{3+}$ ions to $Fe^{2+}$ ions,
(B) a semipermeable layer, which is permeable for iron ions, but which is impermeable for polypeptides having a molecular weight of more than 300 kDa and
(C) a zeolite suitable for adsorbing the iron ions which is suspended in water or an aqueous buffer;

(ii) placing the semipermeable layer between the ferritin-containing fluid and the zeolite so that the ferritin-containing fluid does not get in direct contact with the zeolite; and
(iii) incubating the arrangement obtained from step (ii) under conditions that allow migration of the iron ions through the membrane and adsorption thereof to the zeolite.

The method is an *in vitro* method, i.e., a method conducted outside the human or animal body.

The present invention further relates to a method for preparing iron-depleted ferritin, wherein preferably less than a fourth of the iron-binding sites is occupied by iron, in particular apoferritin, wherein said method comprises the steps (i) to (iii) as laid out above.

Iron-depleted ferritin can be used in a similar indication as ceruloplasmin to catch excesses from iron. This will reduce the oxidative stress and, therefore, the tissue damage in inflammation sites.

Further, the present invention relates to a method for reducing the concentration of one or more species of inorganic ions in a fluid, wherein said method comprises the following steps:

(i) providing:

(A) the body fluid containing ferritin with an addition of one or more reducing agents reducing $Fe^{3+}$ ions to $Fe^{2+}$ ions,
(B) a semipermeable layer, which is permeable for the one or more species of inorganic mono, bi-, tri- or polyvalent ions (preferably including iron ions), but which is essentially impermeable for polypeptides having a molecular weight of more than 10 kDa and
(C) a zeolite suitable for adsorbing the inorganic ions which is it is suspended in water or an aqueous buffer;

(ii) placing the semipermeable layer between the ferritin-containing fluid and the zeolite so that the ferritin-containing fluid does not get in direct contact with the zeolite; and
(iii) incubating the arrangement obtained from step (ii) under reducing conditions that allow the formed bivalent ions to migrate from the ferritin into the buffer and
(iv) allow than the migration of the one or more species of inorganic ions through the membrane and adsorption thereof to the zeolite.

Iron-depleted ferritin, in particular apoferritin, is capable to be used for the treatment of iron overloads in patients. Iron overload and accumulations are one of the main causes for multiple diseases and adverse effects in patients, including oxidative stress, tissue damage in chronic inflammations, neuronal diseases like Parkinson's and others.

The ferritin is isolated from human plasma and then depleted of its stored iron ions by the usage of zeolites and eventually reducing agents. A reducing agent may mediate the electron transfer by reducing the iron ions (often $Fe^{3+}$ and $Fe^{2+}$) in the ferritin to water soluble iron ions (often $Fe^{2+}$). Zeolites are provided behind a semipermeable layer (e.g., a filter, a filter membrane, an osmotic membrane or the like), in a buffer containing ions with a lower selectivity for the zeolite than iron. Iron-depleted ferritin, in particular apoferritin, is capable to remove the excess iron from the site of inflammation, allowing the body to heal the inflammation and restore iron homeostasis.

Surprisingly, it was found that some special zeolites can be used to decrease the concentration of iron ions, from a body fluid, such as plasma, in sufficient way. In combination with a reduction (e.g., using NAD or NADH), such decrease of the concentration of inorganic ions, such as iron ions, can be obtained either within the solution or in two solutions, separated by a semipermeable membrane whereby one compartment contains the ferritin-containing solution like plasma or any intermediate from the plasma fractionation plus the reduction reagent and the second compartment contains a suspension of zeolites. To support the reductive mobilization of iron atoms from the ferritin solution, voltage can be applied to the solution to guarantee a constant reduction and compensate for unwanted oxidations by the oxygen molecules of the environmental air (Koochana et al.: Releasing iron from ferritin protein nanocage by reductive method: The role of electron transfer mediator, Biochemica et Biophysica Acta, 2018, 1862:1190-1198).

Using this procedure, an iron-free ferritin can be produced which can be used later on as drug for the treatment of acute or chronic inflammations in several tissues like brain, gastro-intestinal tract, lung, heart, liver, kidney, bones, etc. As used herein, iron ions may preferably be selected from $Fe^{3+}$ ions and $Fe^{2+}$ ions.

In a preferred embodiment, the method is conducted at conditions reducing the oxidation state of $Fe^{3+}$ [also: Fe(III) ions to $Fe^{2+}$ [also: Fe(II)] ions. Typically, the ferritin-containing fluid contains one or more reducing agents. Preferably, at least one reducing agent is added to the ferritin-containing fluid. Such reducing agent may be any reducing agent suitible for reducing $Fe^{3+}$ ions to $Fe^{2+}$ ions. In other words, preferably, the ferritin-containing fluid contains one or more reducing agents to mediate the electron transfer by reducing the iron ions in the ferritin to water-soluble iron ions (typically $Fe^{2+}$).

In such reduction step, $Fe^{3+}$ ions that are bound to ferritin and/or other proteins and/or $Fe^{3+}$ ions that are unbound, in particular dissolved $Fe^{3+}$ atoms, may be reduced to $Fe^{2+}$ ions. In the case (essentially) no $Fe^{3+}$ ions are present, the one or more reducing agents maintain iron ions as $Fe^{2+}$ ions in an oxidative environment containing oxygen. It will be understood that oxygen is typically present in an aqueous environment. Thus, typically, the environment is oxidative and typically $Fe^{2+}$ ions are oxidized to $Fe^{3+}$ ions when no reducing agent is present.

In a preferred embodiment, the reducing agent is mild enough to maintain the ferritin/apoferritin polypeptide functionality.

In a preferred embodiment, such reducing agent may be selected from the group consisting of the reduced or oxidized form of nicotinamide adenine dinucleotide [NADH (also designated as: $NADH_2$) or NAD], flavin mononucleotide (FMN, also designated as: $FMNH_2$ or riboflavin-5'-phosphate), dithionate, ascorbic acid, and a combination or mixture of two or more thereof (e.g., NADH/FMN). In a NADH/FMN-system, NAFH may serve as the (main) reducing agent, while FMN may serve as electron transfer mediator. The NADH/FMN-system is also described in Koochana et al. (Koochana et al.: Releasing iron from ferritin protein nanocage by reductive method: The role of electron transfer mediator, Biochemica et Biophysica Acta, 2018, 1862:1190-1198).

In a preferred embodiment, such reducing agent may be ascorbic acid and/or the reduced form of nicotinamide adenine dinucleotide (NADH) in combination with flavin mononucleotide (FMN) (i.e., NADH/FMN). In a preferred embodiment, such reducing agent may be (the reduced form of) ascorbic acid.

In a preferred embodiment, a reducing agent may be present in the ferritin-containing fluid in any concentration sufficient for reducing $Fe^{3+}$ ions to $Fe^{2+}$ ions. For example, a reducing agent may be used at a concentration of 0.001 to 1000 mM, 0.005 to 750 mM, 0.01 to 500 mM, 0.02 to 50 mM, 0.03 to 100 mM, 0.04 to 75 mM, 0.05 to 50 mM, 0.1 to 25 mM, 0.5 to 20 mM, 0.5 to 15 mM, 1 to 10 mM, 2 to 9 mM, 3 to 8 mM, 3 to 6 mM, 4 to 6 mM, or 5 mM. Such concentration may represent the concentration of each reducing agent or may be sum of all reducing agents.

In a preferred embodiment, the ferritin-containing fluid contains (the reduced form of) ascorbic acid in a concentration of 0.001 to 1000 mM, 0.005 to 750 mM, 0.01 to 500 mM, 0.02 to 50 mM, 0.03 to 100 mM, 0.04 to 75 mM, 0.05 to 50 mM, 0.1 to 25 mM, 0.5 to 20 mM, 0.5 to 15 mM, 1 to 10 mM, 2 to 9 mM, 3 to 8 mM, 3 to 6 mM, 4 to 6 mM, or 5 mM.

In a preferred embodiment, the ferritin-containing fluid contains (the reduced form of) NADH in a concentration of 0.001 to 1000 mM, 0.005 to 750 mM, 0.01 to 500 mM, 0.02 to 50 mM, 0.03 to 100 mM, 0.04 to 75 mM, 0.05 to 50 mM, 0.1 to 25 mM, 0.5 to 20 mM, 0.5 to 15 mM, 1 to 10 mM, 2 to 9 mM, 3 to 8 mM, 3 to 6 mM, 4 to 6 mM, or 5 mM.

In a preferred embodiment, the ferritin-containing fluid contains (the reduced form of) NADH in a concentration of 0.001 to 1000 mM, 0.005 to 750 mM, 0.01 to 500 mM, 0.02 to 50 mM, 0.03 to 100 mM, 0.04 to 75 mM, 0.05 to 50 mM, 0.1 to 25 mM, 0.5 to 20 mM, 0.5 to 15 mM, 1 to 10 mM, 2 to 9 mM, 3 to 8 mM, 3 to 6 mM, 4 to 6 mM or 5 mM and the FMN in a NADH : FMN molar ratio of between 1000 : 1 and 10 : 1, of between 500 : 1 and 50 : 1, of between 200 : 1 and 70 : 1, of between 150 : 1 and 80 : 1, or (approximately) 100 : 1.

Further examples of reducing agent compositions are provided in the Example section below.

A ferritin-containing fluid may be any fluid that contains ferritin. As describes above, the ferritin-containing fluid may further contain any reducing agent that enables reduction $Fe^{3+}$ ions to $Fe^{2+}$ ions.

Ferritin may be any ferritin. Ferritin may be human, animal, fungal, plant or bacterial ferritin or a combination of two or more thereof. In a preferred embodiment, ferritin is mammalian ferritin. In a preferred embodiment, ferritin is human ferritin. Ferritin comprises at least two bound iron ions (i.e., is loaded with two or more iron atoms). Typically, ferritin comprises at least two, at least five, at least ten, at least 20 bound iron ions, at least 50 bound iron ions or at least 100 bound iron ions.

Human ferritin may comprise a light chain (e.g., such designated as UniProtKB - P02792 (FRIL_HUMAN) or a sequence homologue having a sequence homology of at least 90%, in particular at least 99%, thereof) and a heavy chain (e.g., such designated as UniProtKB - P02794 (FRIH_HUMAN) or a sequence homologue having a sequence homology of at least 90%, in particular at least 99%, thereof) loaded with two or more, typically three or more, five or more, ten or more iron ions, 20 or more iron ions, 50 or more iron ions or 100 or more iron ions.

Human ferritin may also comprise mitochondrial ferritin e.g., such designated as UniProtKB - Q8N4E7 (FTMT_HUMAN) or a sequence homologue having a sequence homology of at least 90%, in particular at least 99%, thereof), loaded with two or more, typically three or more, five or more, ten or more iron ions, 20 or more iron ions, 50 or more iron ions or 100 or more iron ions. Ferritin may also be a globular protein complex comprising a number of (in human ferritin typically 24) protein subunits forming a nanocage with multiple metal-protein interactions.

As used herein, the term "decreasing iron-content of ferritin" may be understood in the broadest sense as diminishing the iron-content (iron-load) of the ferritin in comparison to the iron-content of ferritin before conducting the method. In a preferred embodiment, the iron-content of ferritin is decreased by at least 10% (mol/mol) in comparison to the iron-content of ferritin before conducting the method. In a preferred embodiment, the iron-content of ferritin is decreased by at least 25% (mol/mol) in comparison to the iron-content of ferritin before conducting the method.

In a preferred embodiment, the iron-content of ferritin is decreased by at least 50% (mol/mol) in comparison to the iron-content of ferritin before conducting the method. In a preferred embodiment, the iron-content of ferritin is decreased by at least 75% (mol/mol), by at least 90% (mol/mol), or by at least 95% (mol/mol), in comparison to the iron-content of ferritin before conducting the method. In a preferred embodiment, the iron-content of ferritin is decreased to the essential absence of iron in the ferritin. Then apoferritin is obtained.

Preferably, the method of the present invention leads to iron-depleted ferritin. As used herein, the term "iron-depleted ferritin" may be understood in the broadest sense as ferritin, wherein the iron content is reduced. In a preferred embodiment, the iron-depleted ferritin bears an iron-content that is decreased by at least 10% (mol/mol), at least 25% (mol/mol), or at least 50% (mol/mol), in comparison to the iron-content of ferritin before conducting the method.

In a preferred embodiment, in the iron-depleted ferritin, less than a fourth (1/4) of the iron-binding sites is occupied by iron. In a preferred embodiment, in the iron-depleted ferritin, less than a fifths (1/5), less than a sixth (1/6), less than a seventh (1/7), less than a eighth (1/8), less than a ninth (1/9), or less than a tenth (1/10) of the iron-binding sites is occupied by iron. In a preferred embodiment, in the iron-depleted ferritin is essentially free of iron, i.e., is apoferritin

A ferritin-containing fluid may be any fluid that contains ferritin. It may be of natural origin or may be obtained by heterologous expression.

In a preferred embodiment, the ferritin-containing fluid is a body fluid. The ferritin-containing fluid may be any body fluid. The body fluid may be a fluid from any animal species including humans. It may be obtained from a vertebrate (e.g., mammals including humans, birds, fishes, amphibia, reptiles, etc.) or from an invertebrate (e.g., arthropod (e.g., insects, spiders, crustacea, crustaceans, etc.), Mollusca, etc.).

In a preferred embodiment, the ferritin-containing fluid is obtained from a vertebrate or an invertebrate (e.g, an insect), a plant, a bacterium or an archaea. In a preferred embodiment, the ferritin-containing fluid is a vertebrate, in particular a mammal body fluid. In a preferred embodiment, the body fluid is a human body fluid.

In a preferred embodiment, the ferritin-containing fluid is selected from the group consisting of blood, blood plasma or a fraction thereof, and hemolymph or a fraction thereof. In a preferred embodiment, the body fluid is selected from the group consisting of mammal blood, mammal blood plasma or a fraction thereof. In a preferred embodiment, the body fluid is selected from the group consisting of human blood, human blood plasma or a fraction thereof. In a preferred embodiment, the body fluid is human blood. In a preferred embodiment, the body fluid is human blood plasma or a fraction thereof.

In a preferred embodiment, the ferritin-containing fluid is a unit of stored blood or plasma. In a preferred embodiment, the body fluid is a unit of mammal stored blood or plasma. In a preferred embodiment, the body fluid is a unit of human stored blood or plasma.

In a preferred embodiment, the ferritin-containing fluid is a body fluid selected from the group consisting of blood, blood plasma or a fraction thereof, ascites, liquor cerebrospinalis, or the ferritin-containing fluid is selected from the group consisting of intracellular or extracellular liquids, cell culture fluids (e.g. supernatants of cell cultures), homogenates or organs or tissues, hemolymph or a fraction thereof, and plant extracts. As used herein, cell cultures may be any cell cultures known in the art such as, e.g, vertebrate cell cultures (e.g., human cell cultures, mammalian cell cultures), invertebrate cell cultures (e.g., insect cell cultures), plant cell cultures, fungal cell cultures (e.g., yeast cell cultures) or bacterial cell cultures. In a preferred embodiment, the ferritin-containing fluid is a unit of stored blood or plasma.

A ferritin-containing fluid may be provided by any means. It may be a stored from the plasma fractionation process obtained from a human or animal body (donor) or produced by gene technologies. Storage may be any kind of storage such as, e.g., storage at room temperature (RT), storage in an cool environment (e.g. in a fridge at a temperature in the range of 1 to 10 °C), storage in the frozen state (e.g. in a freezer at a temperature in the range of -25 to -1 °C or -90 °C to -60 °C or in liquid nitrogen at around -196 °C), or storage of a freeze dried state at any temperature, preferably below 30 °C. Accordingly, storage may be at a temperature range of 15 °C to 30 °C, 1 °C to 10 °C, -25 °C to 25 °C, -70 °C to -15 °C, -90 °C to -60 °C, - 200 °C to -80 °C, at around -196 °C or below -196 °C.

Storage may be storage for at least one or more minutes (min), for at least an hour (h), for at least six hours, for at least twelve hours, for at least a day (d), for at least a week, for at least a month, for at least two months, for at least six months, or for at least a year.

Provision of a ferritin-containing fluid freshly obtained from a human or animal body (donor) may be performed by any means. Preferably such provision is conducted by means which do not involve a (severe) health risk for the human or animal. For example, such provision may be blood sampling optionally followed by blood fractioning. Blood sampling may be, for instance venous blood sampling or arterial blood sampling.

Alternatively, or additionally to using one or more reducing agents, also an electrical current may also be used to reduce $Fe^{3+}$ ions to $Fe^{2+}$ ions.

In a preferred embodiment, the method of the present invention further comprises contacting a cathode with the ferritin-containing fluid and an anode with the water or aqueous buffer in which the zeolite is suspended and applying a voltage suitable for reducing $Fe^{3+}$ ions to $Fe^{2+}$ ions and/or suitable for reducing the one or more reducing agents.

In other words, the method of the present invention may further optionally comprise inert electrodes in the solution to apply a certain voltage to constantly reduce the electron mediators, that are sufficient to reduce (party oxidized) iron ions.

The applied voltage may be any voltage suitable for the purpose of reducing $Fe^{3+}$ ions to $Fe^{2+}$ ions. Preferably, the applied voltage is mild enough to prevent the proteins, in particular ferritin, from being denaturized. Thus, the reducing agent is preferably mild enough to maintain the ferritin/apoferritin polypeptide functionality.

In a preferred embodiment, the applied voltage is in the range of from 0.1 to 20 V (volts). In a preferred embodiment, the applied voltage is in the range of from 0.2 to 15 V, of from 0.3 to 10 V, of from 0.4 to 8 V, of from 0.5 to 6 V, of from 1 to 6 V, of from 2 to 5 V, of from 1 to 4 V, of from 2.5 to 4 V, or such as (approximately) 3 V or approximately) 3.5 V. In a preferred embodiment, in order to diminish undesired electrolysis side effects, the applied current is rather low. In a preferred embodiment, the applied current is below 100 mA (milliamperes), preferably below 50 mA, between 0.01 and 20 mA, between 0.1 and 10 mM, or between 1 and 5 mA.

In a preferred embodiment, one or more reducing agents may be used in combination with applying an electrical current. In a preferred embodiment, the reducing agent may be selected from the group consisting of (the reduced forms of) ascorbic acid, NADH, FMN, dithionite, and combinations of two or more thereof and the voltage and current are as described above. Examples are provided in the Example section below.

It will be understood that one or more of the one or more reducing agents may optionally also migrate through the semipermeable layer from the ferritin-containing fluid to the suspended zeolite and vice versa. Thus, the one or more reducing agents may distribute in the liquids of the whole device.

In a preferred embodiment, one or more of the one or more reducing agents may be removed from the ferritin-containing fluid after its iron content is decreased. This may optionally be performed in a further step.

Optionally, the ferritin-containing fluid may comprise one or more further ingredients that may support reducing activity. Such further ingredients may, for instance, be one or more chaotropic agents. In other words, the ferritin-containing fluid may comprise one or more chaotropic substances to reduce the interactions between the sub-units of ferritin to allow an easier movement of the electron mediator into the ferritin to access the ferrihydrite core. A chaotropic agent may make the iron ions (in particular $Fe^{3+}$) bound to proteins such as ferritin more accessible to the one or more reducing agents. Optionally, a chaotropic agent may open the channels for the reducing agents (also: electron mediators) to access the ferrihydrite core of the ferritin and to reduce the incorporated iron ions (in particular $Fe^{3+}$).

In a preferred embodiment, the ferritin-containing fluid further comprises one or more chaotropic agents decreasing interactions between ferritin sub-units and/or releasing iron ions from ferritin.

In a preferred embodiment, such chaotropic agent is selected from the groups consisting of urea, n-butanol, ethanol, guanidinium chloride, barium salts, thiocyanates, perchlorates, lithium acetate, magnesium chloride, magnesium sulfate, phenol, 2-propanol, sodium dodecyl sulfate, thiourea, and combinations of two or more thereof. In a preferred embodiment, such chaotropic agent is urea.

A chaotropic agent may be used in any concentration suitable for support reducing activity. For instance, a chaotropic agent may be used in a concentration of from 0.01 to 10 M (molar), of from 0.1 to 9 M, of from 0.5 to 8 M, or of from 1 to 5 M. This concentration may be the concentration of one or each chaotropic agent or may be the concentration of the sum of chaotropic agents. Examples for uses of chaotropic agents are provided in the Example section below.

It will be understood that the chaotropic agent may optionally also migrate through the semipermeable layer from the ferritin-containing fluid to the suspended zeolite and vice versa. Thus, the chaotropic agent may distribute in the liquids of the whole device.

In a preferred embodiment, the chaotropic agent may be removed from the ferritin-containing fluid after its iron content is decreased. This may optionally be performed in a further step.

The zeolite may have any properties suitible for adsorbing iron ions. In a preferred embodiment, the zeolite is also suitible for further adsorbing one or more further inorganic ions.

The one or more species of inorganic ions may be any inorganic ions known in the art that may be comprised in a ferritin-containing fluid. In a preferred embodiment, the inorganic ions in the sense of the present invention have an ionic weight of not more than 200 Da, in particular not more than 100 Da or not more than 50 Da. In a preferred embodiment, the inorganic ions in the sense of the present invention are one or more species of cations. In the context of the present invention, an inorganic ion may have any valency. An inorganic ion may be a mono-, bi-, tri- or polyvalent inorganic ion. In a preferred embodiment, the one or more species of inorganic ions have a valency of two or more. In a preferred embodiment, the one or more species of inorganic ions are bi-, tri- or polyvalent inorganic ions. In a more preferred embodiment, the one or more species of inorganic ions are bi- or trivalent inorganic ions. In a preferred embodiment, the inorganic ions in the sense of the present invention are one or more species of cationic metal ions. In a preferred embodiment, the inorganic ions in the sense of the present invention are one or more species selected from the group consisting of alkaline earth metals (group 2 of the periodic table of elements), earth metals (group 13 of the periodic table of elements, and a transition metal cation, each having a valency of II or III.

In a preferred embodiment, the zeolite is also suitible for further adsorbing one or more further inorganic ions selected from the group consisting of zinc ions, calcium ions, aluminum ions, plutonium ions, cesium ions, beryllium ions, copper ions, zinc ions, and calcium and zinc ions. In a particularly preferred embodiment, the inorganic ions in the sense of the present invention are one or more species naturally found in ferritin-containing fluid without xenobiotic influences.

In a particularly preferred embodiment, the inorganic ions in the sense of the present invention are one or more species selected from the group consisting of calcium, zinc, magnesium, aluminum, nickel, iron, and copper ions.

In a particularly preferred embodiment, the one or more species of inorganic ions comprise or are ions selected from the group consisting of calcium ions, zinc ions, and calcium and zinc ions. In a highly preferred embodiment, the one or more species of inorganic ions comprise or consist of calcium ions. In a preferred embodiment, the inorganic ions consist of calcium ions. In a preferred embodiment, the one or more species of inorganic ions comprise or are zinc ions. In a preferred embodiment, the inorganic ions are zinc ions. In a preferred embodiment, the one or more species of inorganic ions comprise or consist of calcium and zinc ions. In a preferred embodiment, the inorganic ions consist of calcium and zinc ions.

In an alternative preferred embodiment, the inorganic ions in the sense of the present invention are one or more species of anions. In an alternative preferred embodiment, the inorganic ions in the sense of the present invention are one or more species of anions selected from the group consisting of carbonate ($CO_3^{2-}$), and sulfate ($SO_4^{2-}$).

The zeolite may be any zeolite in the art that can interact with the one or more species of inorganic ions of interest, in particular iron. In a preferred embodiment, the zeolite adsorbs at least parts of the one or more species of inorganic ions of interest, in particular iron. In a preferred embodiment, the zeolite is an aluminosilicate zeolite.

The zeolite may have any form. Typically, the zeolite is a solid that may be either a monolith or may be fragmented.

In a preferred embodiment, the zeolite is provided as a powder, in particulate form or as a paste.

In a preferred embodiment, the zeolite is provided as a powder. In a preferred embodiment, the zeolite is provided in particulate form.

As used herein, the term "powder" may be understood in the broadest sense as any solid particles having a mean small diameter, such as <10 $\mu$m (e.g., determined by Laser diffraction analysis such as a Malvern Metasizer).

As used herein, the term "in particulate form" may be understood in the broadest sense as any solid particles having a mean diameter between 0.01 mm and 10 mm, 0.1 to 5 mm, or 0.5 to 2 mm (e.g., determined by sieve analysis or microscopic or macroscopic measurements). A particulate form may have any shape. It may be (essentially) spherical, broken or crystalline. In a preferred embodiment, the zeolite particles are (essentially) spherical. In a preferred embodiment, the zeolite particles are (essentially) spherical and have a mean particle diameter of 0.1 to 5 mm (e.g., determined by sieve analysis or microscopic or macroscopic measurements).

In a preferred embodiment, the zeolite bears pores that may incorporate and at least partly capture the one or more species of inorganic ions of interest, in particular iron.

In a preferred embodiment, the zeolite has a pore size in the range of 140 to 600 pm. In a preferred embodiment, the zeolite has a pore size in the range of 300 to 500 pm. In a preferred embodiment, the zeolite has a pore size in the range of 350 to 450 pm, 375 to 425 pm, 380 to 420 pm, 390 to 410 pm or (around) 400 pm.

In one embodiment, the zeolite is particulate and has a mean particle size in the range of from 0.1 to 5 mm, 0.2 to 4 mm, 0.5 to 3.5 mm, 1 to 3 mm, or 1.5 to 2.5 mm.

For instance, the zeolite may be UOP MOLSIV® TE 143 R (UOP, type A, sodium form, pore size 4 angstrom $(4 \times 10^{-10}$ m), 1.4-2.3 mm (mean approx. 2.0 mm), powder density > 745 kg/m$^3$);

In a preferred embodiment, the zeolite is provided in a monovalent ion-loaded loaded form. In a preferred embodiment, the zeolite is provided in an alkaline metal loaded form such as, e.g., as sodium- and/or potassium-loaded zeolite.

It will be understood that the person skilled in the art may adapt pore size to the one or more species of inorganic ions of interest, in particular iron. In case the ions have a larger diameter, also the pore size may be chosen to be larger. In case the ions have a smaller diameter, also the pore size may be chosen to be smaller.

The zeolite may be used in a suspension or may be used in dry state. In a preferred embodiment, the zeolite is suspended in an aqueous buffer. As used throughout the present invention, the buffer is preferably a pharmaceutically acceptable buffer such as, e.g., phosphate buffered saline (PBS).

As used herein, pharmaceutically acceptable may be understood in the broadest sense as any compound that can be used in a pharmaceutical context, i.e., is (essentially) non-toxic in the used concentration range.

This can mean, but does not necessarily mean, that the buffer is officially approved for pharmaceutical uses (e.g., by the US and/or European Pharmacopeia, in particular each in the actual version at the filing date).

The zeolite may be suspended in an aqueous buffer prior to being used in the context of the present invention.

In a preferred embodiment, the zeolite is provided as a powder, in particulate form or as a paste and has a pore size in the range of 140 to 600 pm, in particular 300 to 500 pm. In a preferred embodiment, the zeolite is provided as a powder or in particulate form and has a pore size in the range of 300 to 500 pm.

As the zeolite preferably has a well-defined structure, the pore size is preferably well-defined. The pore size may also be designated as mean pore size. As used herein, the pore size may be determined by commons means in the art. As the zeolite preferably has a well-defined structure, the pore size may be directly obtained from the chemical structure. The pore size may refer to the hydrodynamic radius. In a preferred embodiment, the pores of the zeolite have (essentially) radial openings. In a preferred embodiment, the pores of the zeolite have (essentially) cylindrical pores. In case the pores of the zeolite do not have radial openings, the pore size preferably refers to the smallest diameter of the pore opening.

In another preferred embodiment, the pore size may also be determined as the specific surface area (SSA). Alternatively, also inverse size exclusion chromatography may be used for determining the pore size. Pore size may be determined as described in Chapter "Porosity and its Measurement" of Espinal in "Characterization of Materials", edited by Elton N. Kaufmann, 2012, John Wiley & Sons, Inc.

In a preferred embodiment, the zeolite is provided as a powder, in particulate form or as a paste suspended in an aqueous buffer. In a preferred embodiment, the zeolite has a pore size in the range of 140 to 600 pm, in particular 300 to 500 pm and is suspended in an aqueous buffer. In a preferred embodiment, the zeolite is provided as a powder, in particulate form or as a paste suspended in an aqueous buffer and has a pore size in the range of 140 to 600 pm, in particular 300 to 500 pm.

The semipermeable layer is (essentially) impermeable for polypeptides having a molecular weight of more than 300 kDa (kilodaltons). As used herein, the term "essentially impermeable" in the context of polypeptides having a molecular weight of more than 300 kDa may be understood in the broadest sense in that polypeptides having a molecular weight of more than 300 kDa do not or nearly not pass through the semipermeable layer. Accordingly, when a certain volume ferritin-containing fluid containing such polypeptides is subjected to one side of the semipermeable layer and a comparable volume of a buffer free of polypeptide is subjected to one side of the semipermeable layer, after incubation at room temperature for 24 hours, the buffer preferably does not contain more than 25% (mol/mol), more preferably does not contain more than 10% (mol/mol) or 5% (mol/mol), in particular does not contain more than 1% (mol/mol) of the concentration of polypeptides having a molecular weight of more than 40 kDa of the initial concentration of the ferritin-containing fluid.

In a preferred embodiment, the semipermeable layer has a cutoff for polypeptides in the range of 1 to 300 kDa, 2 to 250 kDa, 2 to 200 kDa, 2 to 150 kDa, 2 to 100 kDa, 2 to 50 kDa, 2 to 45 kDa, 20 to 40 kDa, 25 to 35 kDa, or 5 to 15 kDa and/or where the semipermeable layer is an osmosis membrane, potentially even selective for certain ions, in particular iron ions. For instance, a semipermeable layer (filter membrane) in a tube/flask such as, e.g., a CentriPrep (e.g., YM-30) (Merck Millipore, 10 kDa cutoff, 15 mL volume) may be used.

In a preferred embodiment, the semipermeable layer is (essentially) impermeable for polypeptides having a molecular weight of more than 30 kDa, of more than 20 kDa, of more than 10 kDa, of more than 5 kDa, or of more than 2 kDa.

The semipermeable layer is permeable for the one or more species of inorganic ions, in particular iron ions. As used herein, the term "permeable" may be understood in the broadest sense in that such inorganic ions may efficiently pass through the semipermeable layer. Accordingly, when a certain volume ferritin-containing fluid containing such ions is subjected to one side of the semipermeable layer and a comparable volume of a buffer free of such ions, after incubation at room temperature for 24 hours, the buffer preferably contains more than 25% (mol/mol), more than 50% (mol/mol), or 75% (mol/mol), of the concentration of the respective one or more species of inorganic ions, in particular iron ions, of the initial concentration of the ferritin-containing fluid.

In a preferred embodiment, the semipermeable layer has a cutoff for polypeptides in the range of below 1 kDa, below 2 kDa, below 5 kDa, below 10 kDa, below 20 kDa, below 30 kDa, below 40 kDa, or below 45 kDa.

In a preferred embodiment, the semipermeable layer has a cutoff for polypeptides in the range of above 1 kDa, above 2 kDa, above 5 kDa, above 10 kDa, above 20 kDa, above 30 kDa, above 40 kDa, or above 45 kDa.

In a preferred embodiment, the semipermeable layer has a cutoff for polypeptides in the range of 2 to 45 kDa, 20 to 40 kDa, 5 to 15 kDa, or 25 to 35 kDa.

A semipermeable layer may be a membrane or a more solid material. It may have any thickness suitable for the method of the present invention. It may, for example have a thickness in the range of 1 to 50 $\mu$m, 25 to 100 $\mu$m, 50 to 1000 $\mu$m, or 0.5 to 2 mm. The semipermeable layer may form part of a device usable in the context of the present invention.

In a preferred embodiment, the semipermeable layer forms part of a device selected from the group consisting of

a filter, in particular a filter selected from the group consisting of a centrifugation filter, a filter for dead-end filtration under standard pressure, high pressure or vacuum, and a cross-flow filtration filter;
a dialysis device, in particular a dialysis device selected from the group consisting of a dialysis membrane, a dialysis tube, and a dialysis bag; and a hollow fiber.

Such devices are commercially available. The method may be conducted in an on-line procedure, wherein the ferritin-containing fluid passes under reducing conditions by the semipermeable layer and, thus, the zeolite or may be conducted in a dead-end (i.e., batch) procedure wherein a batch of the ferritin-containing fluid is contacted with the semipermeable layer and, thus, the zeolite, for a defined time.

In a preferred embodiment, the method is conducted in an on-line (also designable as in-line) procedure, preferably wherein the ferritin-containing fluid flows alongside the semipermeable layer, in particular wherein the method is conducted on-line within an apheresis or blood donation procedure.

In a preferred embodiment, in particular when conducted as an on-line procedure, step (iii) of incubating is conducted for at least 1 s (second), at least 10 s, or at least 20 s.

In a preferred embodiment, in particular when conducted as a dead-end (i.e., batch) procedure, step (iii) of incubating is conducted for at least 5 min, at least 10 min, or at least 20 min.

In a preferred embodiment, the step (iii) of incubating is conducted until the concentration of iron ions in the ferritin-containing fluid is reduced by at least 25% (mol/mol) in comparison to the concentration contained in the ferritin-containing fluid before conducting said method.

In a preferred embodiment, the step (iii) of incubating is conducted until the concentration of the one or more species of inorganic ions, in particular iron ions, in the ferritin-containing fluid is reduced by at least 50% (mol/mol) or at least 75% (mol/mol) in comparison to the concentration contained in the ferritin-containing fluid before conducting said method.

The method of the present invention may be conducted as the sole method for reducing the concentration of one or more species of inorganic ions in a ferritin-containing fluid or may be combined with one or more further means usable for this purpose.

In a preferred embodiment, the method further comprises the addition of one or more complexing agents which complex the one or more of the one or more species of ions, in particular including iron.

Such complexing agent may be any agent that is suitable for complexing the one more species of inorganic ions of interest, in particular iron.

In the context of iron ions, complexing agents may, for example, be selected from citrate and 2,2-bipyridine. Accordingly, in a preferred embodiment of the present invention, a complexing agent is citrate, 2,2-bipyridin, or a combination thereof.

In a preferred embodiment, the method further comprises the addition of one or more agents which form precipitated with the one or more of the one or more species of inorganic ions, in particular iron ions.

For example, higher concentrations of carbonates and/or phosphate ions may precipitate with cations, such as calcium cations.

As indicated above, the ferritin-containing fluid obtainable from the method of the present invention bears particularly beneficial properties. It has a reduced concentration of one or more species of inorganic ions of interest and does also not comprise large amounts of complexing agents.

Accordingly, a further aspect of the present invention relates to a ferritin-containing fluid having a reduced concentration of one or more species of inorganic ions, in particular iron ions, obtainable or obtained from a method of the present invention. After using the method invented here, an essentially iron-depleted, in particular an (essentially) iron-free, ferritin will be obtained.

The iron-depleted ferritin may be obtained in dried or freeze-dried form or may be comprised in a fluid. Accordingly, a further aspect of the present invention relates to a fluid which contains iron-depleted ferritin. Optionally, such fluid may be a pharmaceutical composition.

In particular in the context of therapeutic means, for administration, the iron-depleted ferritin may be comprised in a pharmaceutical composition, i.e., is combined with least one pharmaceutically acceptable carrier. The terms "pharmaceutical composition" and "pharmaceutical formulation" may be understood interchangeably.

As used herein, the terms "pharmaceutically acceptable carrier", "pharmaceutically acceptable excipient", "carrier" and "excipient" may be understood interchangeably in the broadest sense as any substance that may support the pharmacological acceptance of the iron-depleted ferritin. Such pharmaceutical composition may be ready to use and may preferably be a liquid formulation, in particular an injection portion. The storage form may also be liquid but may also be a dried form (e.g. a powder such as a powder comprising dried or freeze-dried the iron-depleted ferritin) or may be a paste or syrup or the like. Optionally, a dried form, paste or syrup may be dissolved or emulsified prior to being administered to the patient.

A pharmaceutically acceptable carrier may exemplarily be selected from the list consisting of an aqueous buffer, saline, water, dimethyl sulfoxide (DMSO), ethanol, vegetable oil, paraffin oil or combinations of two or more thereof. Furthermore, the pharmaceutically acceptable carrier may optionally contain one or more detergent(s), one or more foaming agent(s) (e.g., sodium lauryl sulfate (SLS), sodium doceyl sulfate (SDS)), one or more coloring agent(s) (e.g., food coloring), one or more vitamin(s), one or more salt(s) (e.g., sodium, potassium, calcium, zinc salts), one or more humectant(s) (e.g., sorbitol, glycerol, mannitol, propylenglycol, polydextrose), one or more enzyme(s), one or more preserving agent(s) (e.g., benzoic acid, methylparabene, one or more antioxidant(s), one or more herbal and plant extract(s), one or more stabilizing agent(s), one or more chelating agents (e.g., ethylenediaminetetraacetic acid (EDTA), and/or one or more uptake mediator(s) (e.g., polyethylene imine (PEI), a cell-penetrating peptide (CPP), a protein transduction domain (PTD), an antimicrobial peptide, etc.).

A further aspect, which is not covered by the present invention, relates to a dosage unit of the pharmaceutical composition usable in the context of the treatment or prevention of the present invention, such as a single dose container or a multiple dosage form.

It will be understood that the definitions and preferred embodiments as laid out in the context of the method herein also *mutatis mutandis* apply to the obtainable or obtained iron-depleted ferritin.

Iron-depleted ferritin may be used for numerous purposes. A further aspect refers to iron-depleted ferritin, wherein preferably less than a fourth of the iron-binding sites is occupied by iron, preferably obtained from a method of the present invention, for use in a method of treating an acute or chronic inflammation, in particular wherein the inflammation site is suffering from or is at risk of suffering from oxidative stress associated with an excess of iron ions. Iron-depleted ferritin may also be used for scientific research studies.

In this context, the iron-depleted ferritin may be administered by any means (e.g., intravenously, intraarterially, orally, etc.).

In one preferred embodiment, the iron-depleted ferritin is administered to the patient systemically. **In** another preferred embodiment, the iron-depleted ferritin is administered locally to an inflammation site (inflammatory lesion).

In a preferred embodiment, the iron-depleted ferritin is administered to the patient by an administration route selected from the group consisting of intravenous (i.v.), intraarterial (i.a.), intracranial (i.c.), intraperitoneal (i.p.), intramuscular (i.m.), and subcutaneous (s.c.) injection), in particular by an administration route selected from the group consisting of intravenous (i.v.), intramuscular (i.m.), and subcutaneous (s.c.) injection). Alternatively or additionally, the pharmaceutical composition may also be suitable for other routes of administration such as, e.g., nasal or transdermal administration.

Iron-depleted ferritin, wherein less than a fourth of the iron-binding sites is occupied by iron, preferably obtained from a method of the present invention, may be used in a method of treating or preventing a neurodegenerative disease, in particular a neurodegenerative disease selected from the group consisting of Parkinson's disease, Alzheimer's disease, Huntington's disease, and amyotrophic lateral sclerosis. A neurodegenerative disease may also be a prion-associated disease (prion disease) such as, e g., Creutzfeldt-Jakob disease or bovine spongiform encephalopathy (BSE).

Iron-depleted ferritin, wherein less than a fourth of the iron-binding sites is occupied by iron, preferably obtained from a method of the present invention, may be used in a method of treating or preventing cancer (e.g., breast cancer).

In these contexts, the iron-depleted ferritin may be administered by any means (e.g., intravenously, intraarterially, orally, etc.). In a preferred embodiment, the iron-depleted ferritin is administered locally to an inflammation site (inflammatory lesion).

A still further aspect, which is not covered by the present invention, refers to a method for reducing the concentration of one or more species of inorganic ions, in particular iron ions, in a body fluid, after intoxication, wherein said method is conducted in accordance with the method of the present invention, preferably wherein said method further comprises using a dialysis procedure, in particular a peritoneal dialysis procedure for detoxification.

It will be understood that the definitions and preferred embodiments as laid out in the context of the method herein also *mutatis mutandis* apply to the medical und und and non-medical uses and methods.

Iron-depleted ferritin may also be used in medicinal, cosmetic, consumer good, food, or beverage compositions, herein for instance as a preservative and/or as a replacement for chelators.

The method of the present invention may be used for replacement of ions. In this context, the method of the present invention for decreasing iron content of ferritin may be conducted as laid out herein.

Then, in a further step, alternative ions (preferably bi- or trivalent ions) may be added to the iron-depleted ferritin. Alternatively or additionally, the zeolite suitable for adsorbing the iron ions is preloaded with the one or more non-iron ions to be included in the ferritin instead of iron.

The invention is further illustrated by the figures, examples and claims.

## Brief Description of the Figures

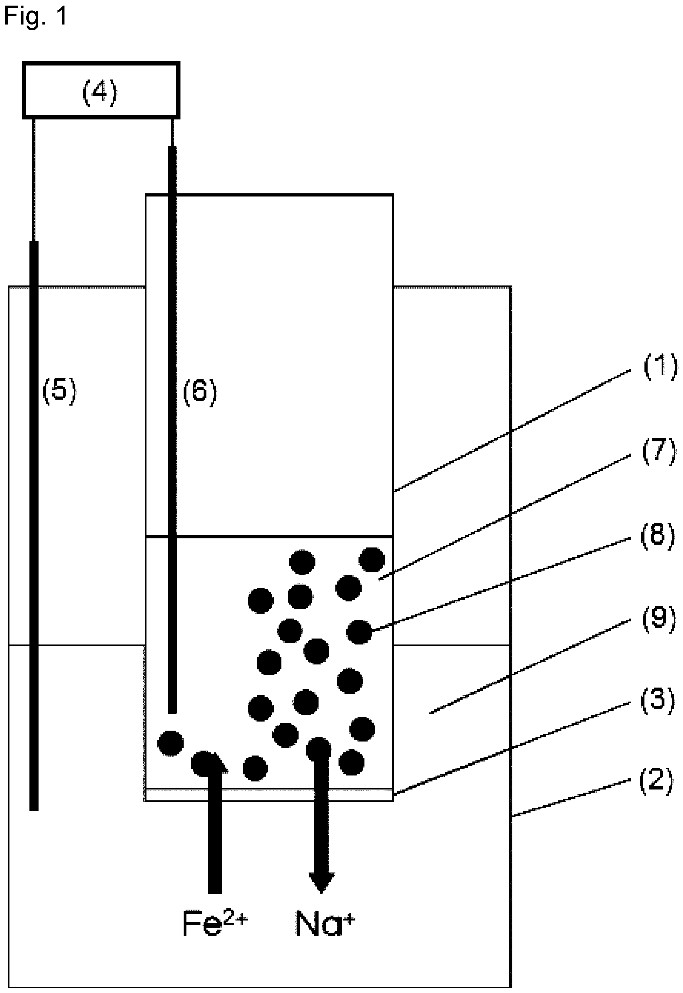

**Figure 1** shows a schematic setup of an electrochemical cell (galvanic cell) usable for the reduction of iron(III) ions to iron(II) ions (and optionally vice versa) ($Fe^{3+} + e^- = Fe^{2+}$). Herein, an inner column (1) in placed into an outer column (main column) (2). (1) and (2) are interconnected by a semipermeable layer (filter membrane) (3). A power supply (power unit) (4) is electrically connected with a cathode (-) (5) and an anode (+) (6). The cathode (5) is placed into the volume of the outer column (2). The anode (6) is placed into the volume of the inner column (1). The volume of the inner column (1) is filled with in PBS (7) with suspended zeolite beads (8). The volume of the outer column (1) is filled a ferritin solution (9).

**Figure 2** shows a photographic image of the electrochemical cell (Bio-Rad electrophoresis chamber).

**Figure 3** shows a photographic image of the electrochemical cell (Bio-Rad electrophoresis chamber) ice-cooled.

**Figure 4** shows comparable setups of devices for investigating the balance between osmotic pressure and electrical potential further. In one setup, the cathode is inserted in the ferritin solution and the anode in the zeolite suspension (A). In another setup, the anode is located in the ferritin solution and the cathode in the zeolite suspension (B). In a still further setup, both electrodes (cathode and anode) are located in the ferritin solution (C). For comparison, in a further setup, no electrodes are used (D).

**Figure 5** shows the absorbance spectrum of a Fe-2,2-bipyridin complex (A) and a NADH/FMN system.

## Examples

Iron removal was realized after reduction of the ferritin-intern iron by NAD or NADH or other chemicals. The reduced iron ions were released from the ferritin into the buffer. The released iron ions were shown to be capable and prevented from re-entering the ferritin molecule by the usage of zeolites to replace them with ions that cannot be incorporated by the ferritin molecule, like sodium.

The zeolites were added behind a semipermeable layer (filter membrane) in a buffer containing monovalent ions, preferably PBS. It was shown that this method allows reduction and finally depletion of the stored iron atoms in the ferritin, allowing the generation of apo-ferritin in a preparative scale.

In experiments, zeolites reduced the iron-content from a ferritin solution without any additional reducing agents (including such acting as electron transfer mediators) or applied voltage, from 0.25 g/L to 0.22 g/L.

## Used Materials

*Devices*

Microtiter plate (polystyrene, clear, flat-bottom);
Falcon tubes (50 mL reaction vessels);
Semipermeable layer (filter membrane) in a tube/flask: CentriPrep YM-30 (Merck Millipore, 10 kDa cutoff, 15 mL volume);
Invitrogen electrophoresis chamber;
Power supplies: Bio-Rad gel-electrophoresis power supply and Voltcraft power supply;
Electrochemical cell: Bio-Rad electrophoresis chamber;
Magnets: neodymium magnet cubes (12 mm diameter);
Reagent reservoir: plastic dish for being filled with protein solution;
MiniMACS column: MACS (Magnetic Cell Separation, Miltenyi Biotech);
Centrifugal columns: 300K NanoSep Filter; and
Sterile filter: 0.22 $\mu$m filter.

*Reagents*

Ferritin: purified ferritin protein;
Ferritin solution; aqueous solution containing ferritin in water;
Fresh-frozen plasma (cryo-poor plasma); and
PCC-depleted (prothrombin complex concentrate-depleated) cryo-poor plasma (initial feed prior to Cohn fractionation).
PBS (Phosphate Buffered Saline);

Reducing agents:   Ascorbic acid, NADH (nicotinamide adenine dinucleotide disodium salt), and FMN (flavin mononucleotide);
Chelating agents:   Citrate, and 2,2-bipyridine;

Urea.
Zeolite: UOP MOLSIV® TE 143 R (UOP, type A, sodium form, pore size 4 angstrom ($4 \times 10^{-10}$ m), 1.4-2.3 mm (mean approx. 2.0 mm), >745 kg/m$^3$);
TMB (3,3',5,5'-Tetramethylbenzidine);
$FeCl_2$ (iron(II) chloride used as tetrahydrate);
$FeCl_3$ (iron(III) chloride); and
Ferene® complex of the Siemens assay.

## Example 1

## Determining the detection limit of $Fe^{2+}$ and $Fe^{3+}$ ions in TMB solution

*Method*

Each 1 ml of $FeCl_2$ (1.2 mg/mL) and 1 mL of $FeCl_3$ (1 mg/mL) were diluted in 1:2 dilution steps. 50 $\mu$L of each dilution step were pipetted into a microtiter plate. 100 $\mu$L of TMB were added into each well. After 1h of incubation, the plate was measured at 595 nm.

*Results and Discussion*

Only $Fe^{3+}$ reacted with TMB in the concentrations from 1 mg/L to 0.25 mg/mL. Since ferritin stores $Fe^{3+}$ or oxidizes $Fe^{2+}$ to $Fe^{3+}$, it was shown possible to determine the stored amount of iron.

## Example2

## Removal of Iron Ions by means of Zeolites

**Example 2A**

**Removal of Different Iron Ions**

It was evaluated whether iron ions can be removed using zeolites (UOP MOLSIV® TE 143 R, behind a semipermeable layer (filter membrane, CentriPrep YM-30) in different samples, free or bound to ferritin.

*Samples*

1) 1mg/mL ferritin solution (reference sample);
2) 1 mg/mL ferritin + zeolites (UOP MOLSIV® TE 143 R, behind semipermeable layer (filter membrane));
3) 1 mg/mL ferritin (reduced to pH 5) + zeolites (behind semipermeable layer (filter membrane));
4) 1 mg/mL $FeCl_2$ + zeolites (behind semipermeable layer (filter membrane)); and
5) 1 mg/mL $FeCl_3$ + zeolites (behind o semipermeable layer (filter membrane)).

*Results and Discussion*

1) 454.44 $\mu$mol/L - 0.025 g/L;
2) 385.72 $\mu$mol/L - 0.022 g/L;
3) 391.57 $\mu$mol/L - 0.022 g/L;
4) 1972.33 $\mu$mol/L - 0.11 g/L; and
5) 3541.31 $\mu$mol/L - 0.20 g/L.

The addition of zeolites to the ferritin (behind semipermeable layer (filter membrane)) only reduced the total amount of iron by a small margin in both samples. Even the use of pH to 5 had no significant influence.

Although the initial concentrations were not measured, they were calculated based on the concentrations of the initial $FeCl_2$ and $FeCl_3$ solutions. Fe(II) concentration was more decreased than the concentration Fe(III).

Calculated initial Fe(II) concentration: 0.28 mg/mL
Fe(II) concentration: 0.11 mg/mL
Calculated initial Fe(III) concentration: 0.34 mg/mL
Fe(III) concentration: 0.20 mg/mL

Since both samples with zeolites decreased iron from the ferritin, most of the iron ions were still incorporated into the ferritin molecule. Even the reduction of pH was obviously not having a significant impact on the release of the iron by altering the conformation of the protein. The concentration was higher than the concentration often found in human blood plasma.

The more significant decrease of Fe(II) ions showed that bivalent ions are preferred by the zeolite for incorporation and exchange for other ions. Potentially due to a limited reduction potential in the first part of the experiment, only the few reduced and released Fe(II) were captured by the zeolites, whereas the majority of Fe(III) ions stayed oxidized and incorporated into the ferritin.

**Example 2B**

**Adjustment of Concentrations**

This example has to two main purposes:

(i) To validate whether zeolites (UOP MOLSIV® TE 143 R) alone are able to remove iron from ferritin; and
(ii) to test whether adjusted concentrations, or adjusted proportions between ferritin and zeolites allow a complete removal of bound iron from ferritin.

*Protocol*

The ferritin solution was poured into the outer column of a semipermeable layer (filter membrane)tube (CentriPrep YM-30) with the zeolites and water on the inner column, and were incubated for one day. Both concentrations in the inner and outer column after incubation and the initial concentration were measured.

*Results and Discussion*

Initial iron concentration: 0.328 $\mu$mol/L;
Inner column of semipermeable layer (filter membrane, CentriPrep YM-30) (zeolites): -0.094 $\mu$mol/L;
outer column of the semipermeable layer (ferritin solution): 0.745 $\mu$mol/L.

The iron content in the outer column was higher than the initial sample and within the inner column, where the zeolites are located, no iron could be measured.

It could not be shown that the zeolites are sufficient to effectively remove iron from a ferritin solution on their own. Additional agents seem to be beneficial to support the electron transfer.

**Example 3**

**Optimization of Removal of Iron Ions by means of Zeolites**

**Example 3A**

**Applying a current to ferritin solutions**

It was intended to evaluate whether the release of stored iron could be optimized since zeolites alone may have a desirable capacity for the complete removal of iron ions. It was found that, in particular when stored ferritin is used, a significant portion (often the majority) of iron ions are stored in the Fe(III) form and are beneficially reduced to be effectively released from the ferritin molecule. Thus, it was tested whether the redox-potential of an electric can be used to reduce and release the iron ions.

*Method*

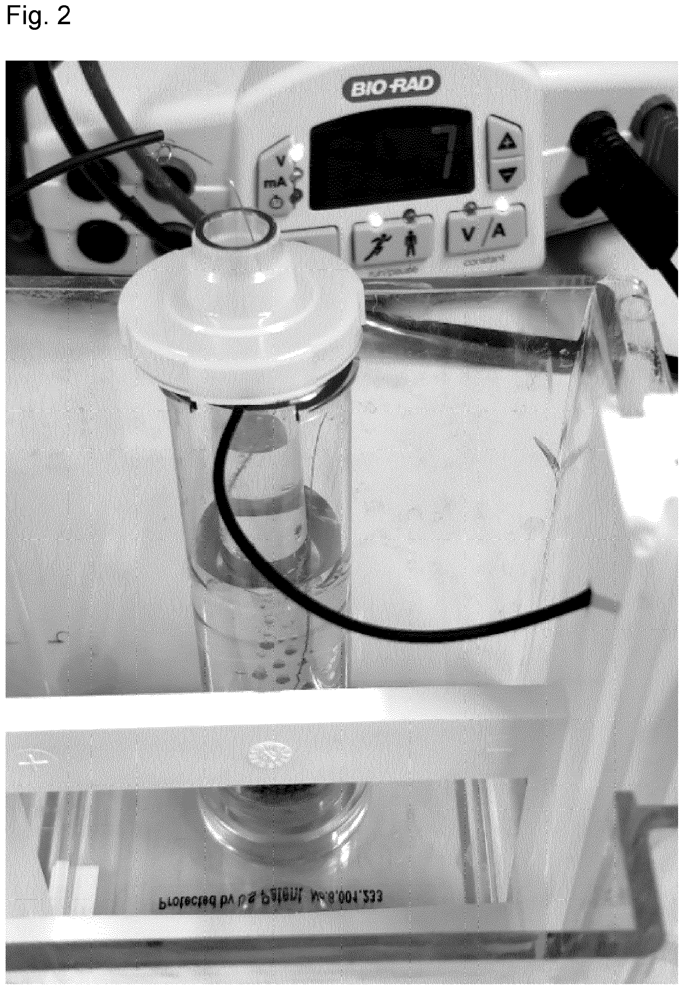

A ferritin solution of 0.2 mg/mL was filled into the outer chamber of an electrochemical cell constructed of a CentriPrep YM-30 tube combined with an Invitrogen electrophoresis chamber. The zeolite (UOP MOLSIV® TE 143 R) suspended in PBS was filled into the inner chamber of said electrolytic cell. The setup is depicted in Figure 1 (schematic) and Figure 2 (photograph).

*Results and Discussion*

The reduction of ferritin was achieved at the cathode and the iron(II) ions were able to move past the membrane and were effectively captured by the zeolites. A constant current of 4 mA (lowest possible current) was used for 45 min to the two solutions and let the device regulate the voltage accordingly.

The resulting voltage fluctuated around 7-12 V. The general color of the iron solution became brighter, indicating a reduction of iron in the solution. A white patch of foam formed at the surface of the liquid in the main column in the combination with brown, floating colloids of in the liquid. Bubbles and foaming were observed.

The measured iron contents resulted in the following values were for the untreated ferritin sample 82.6 $\mu$mol/L, while after applying the current (voltametric treatment), it increased to 118.1 $\mu$mol/L. Unexpectedly the value of our treated sample was higher than the initial sample. It was considered that, potentially, the measured sample contained a surplus of colloids leading to a wrongly high iron value. This was investigated further in the following experiments.

**Example 3B**

**Optimization of the reduction of immobilized iron ions from the ferritin**

Further experiments were performed in order to optimize the reduction of immobilized iron ions from the ferritin.

The following optimizations had to be included:

- A complete Redox-System consisting of NADH and FMN
- A power supply capable for lower constant voltages to prevent any potential damage at the proteins

*Method*

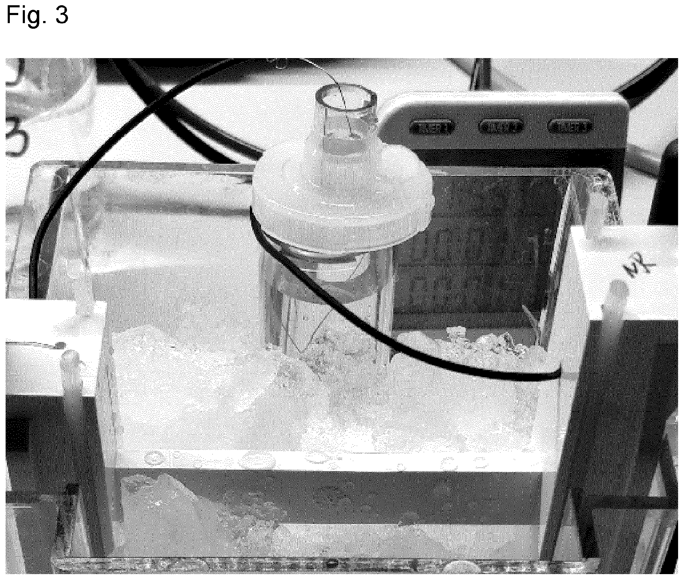

An electrochemical cell was constructed of a CentriPrep YM-30 tube combined with an Invitrogen electrophoresis chamber. The setup is depicted in Figure 1 (schematic) and Figures 2 and 3 (photographs). Around 3 g (surplus) of zeolites (UOP MOLSIV® TE 143 R) were incubated and activated in the inner CentriPrep YM-30 tube in PBS. Preparation of a ferritin sample:

25 mL of a 10 $\mu$g/mL ferritin solution in PBS,
20 mL of PBS, and
5 mL of PBS with 25 mM nicotinamide adenine dinucleotide disodium salt (NADH) and 500 $\mu$M Flavin mononucleotide (FMN).

Ice was added to the electrochemical cell to prevent any overheating of the solutions in the CentriPrep YM-30 tube. Then, by means of a Voltcraft power supply, a voltage of 0.3 mV was applied for 2 hours to the solution. Samples were withdrawn before and after applying the current (voltametric treatment).

In order to investigate the balance between osmotic pressure and electrical potential further, placement of the electrodes was permutated as follows:

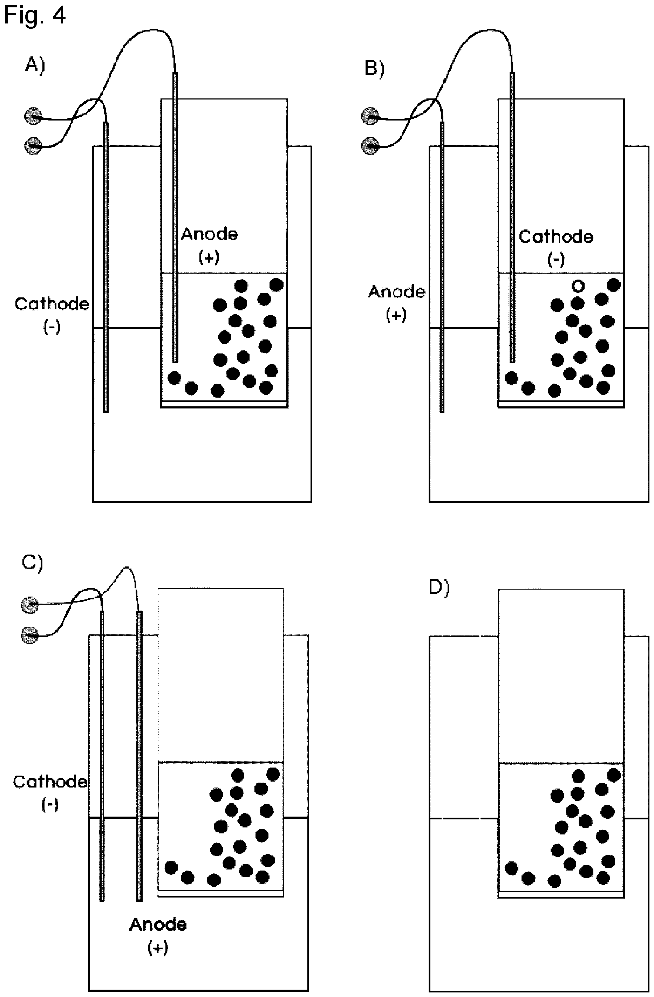

1) Cathode in the ferritin solution and anode in the zeolite suspension (cf. Figure 4A);
2) Anode in the ferritin solution and cathode in the zeolite suspension (cf. Figure 4B);
3) Both electrodes (cathode and anode) in the ferritin solution (cf. Figure 4C); and
4) No electrodes used just incubation (cf. Figure 4D).

*Results and Discussion*

In the contrary to the setup used in Example 3A, no bubbles, foaming or formation of brown colloids were observed. Against expectations, the iron concentrations of the treated samples were higher than the initial concentrations. The results are depicted in Table 1.

**Table 1.** Results for different setups of electrochemical cells

| Setup | Iron concentration [$\mu$mol/L] | |
|---|---|---|
| | untreated | treated |
| Cathode in the ferritin solution and anode in the zeolite suspension (cf. Figure 4A) | 6.443 | 8.136 |
| Anode in the ferritin solution and cathode in the zeolite suspension (cf. Figure 4B) | 7.277 | 8.029 |
| Both electrodes (cathode and anode) in the ferritin solution (cf. Figure 4C) | 8.420 | 9.032 |
| No electrodes used just incubation (cf. Figure 4D) | 7.891 | 9.989 |

It was considered that the increased total iron concentrations may be due to a reduction of volume in the outer column, leading to an increased concentration of iron. This may also be combined with a possible low uptake of iron by the zeolites. It was further considered that, potentially, the buffer system is not suitable for the reduction of iron These findings were investigated further in the following Examples.

**Example 3C**

**Reductive mobilization of iron from ferritin**

It was decided again to test the possible reductive mobilization of iron from ferritin.

*Methods*

An aqueous ferritin solution (10 $\mu$g/mL) solution was tested in two setups, both with the cathode in the ferritin solution and the anode in the inner column with the zeolites. The setup on the device was as depicted in Examples 3A and 3B (Cathode in the ferritin solution and anode in the zeolite suspension (cf. Figures 1 and 4A). Each ferritin sample consisted of 50 ml 0.5x PBS with 5mM NADH and 50 $\mu$M FMN. The first was sample was treated for 24h with a voltage of 0.33 V measured and then treated for another 24 hours at 1 V. The pH of the second sample was decreased to pH 5 was treated for 24 hours at 0.33 V. The voltage was increased to 3.5 V for the second 24 hours incubation. The results are depicted in Table 2.

**Table 2.** Results for different samples and voltages in electrochemical cells each applied for 24 hours

| Sample and voltage | Iron concentration [$\mu$mol/L] |
|---|---|
| Feed (before applying electrochemical treatment) | 6.216 |
| 0.33 V at pH 7.4 | 6.166 |
| 1 V at pH 7.4 | 5.823 |
| 0.33 V at pH 5 | 3.035 |
| 3.5 V at pH 5 | 10.749 |

Only the decrease of the pH to 5 seemed to cause a noticeable decrease of the iron content in the ferritin solution. The reduction of the pH seems to help the release of iron from ferritin.

**Example 4**

**Use of ferromagnetic properties for enriching ferritin**

**Example 4A**

**Attempt of enriching ferritin by means of applying neodymium magnet cubes**

It was evaluated whether the ferromagnetic properties are enough to separate or locally enrich ferritin from the remaining proteins of a solution.

Two ferritin solutions were tested. The first was ferritin in water and the second was ferritin in a high protein solution in cryo-poor plasma. Both solutions were provided in a reagent-reservoirs, where each magnet (neodymium magnet cubes of 12 mm diameter were placed on one side of the reservoir. After 5 hours, two samples were withdrawn from the two sides of the reservoir and their iron contents are measured. Results of different samples are depicted in Table 3.

**Table 3.** Effect of magnets on ferritin - Iron concentration

| Sample | Feed / untreated | Non-magnet side | Magnet side |
|---|---|---|---|
| Ferritin solution in water | 19.646 $\mu$mol/L | 15.811 $\mu$mol/L | 13.370 $\mu$mol/L |
| Cryo-poor plasma | 23.820 $\mu$mol/L | 25.346 $\mu$mol/L | 25.640 $\mu$mol/L |

No influence from the magnet on the ferritin molecules in the solution could be seen.

**Example 4B**

**Attempt of enriching ferritin from cryo-poor plasma by means of applying neodymium magnet cubes with long incubation times**

Example 4A was repeated with a longer incubation time of one day (24 hours). A fresh-frozen plasma solution (cryo-poor plasma) containing ferritin and a bar of neodymium magnets was placed nearby for 24 hours. After incubation the concentrations on both sides of the reservoir, facing towards and distant from the magnet.

While the feed had an iron content of 26.012 $\mu$mol/L, the non-magnet side had an iron content of 40.940 $\mu$mol/L, and the magnet side had an iron content of 41.555 $\mu$mol/L. The values of both sides of the reservoir were both almost identical and higher than the initial value. Although the incubation time was prolonged, no dependence on the proximity to the magnet could be determined. Since the magnetic force drops by the radius to the square, the resulting strength of the magnetic field was too weak to arrange and accumulate the ferritin particles in different solutions.

**Example 5**

**Attempt of enriching ferritin by means of applying strong magnetic forces**

To increase the strength of the magnetic field, MACS (Magnetic Cell Separation) - columns were applied to offer a stronger magnetic field, especially with the close proximity of the magnetic beads and the solution. This system includes a column holding many little ferrite beads, which can be placed into a big magnetic holder. If placed into the holder, the flux density is amplified by a factor around 10,000.

*Methods*

Water containing ferritin and fresh-frozen plasma (cryo-poor plasma) containing ferritin were tested in two different samples.
**Sample 1:** The column was placed into magnet. 5 mL feed were poured onto the MiniMACS column. The flow-through was captured. Then, the column was removed from magnet. 5 mL of PBS were poured onto the column and captured as eluate.
**Sample 2:** The column was placed into magnet. 5 mL feed were poured onto the MiniMACS column. 5 mL PBS were poured onto the column. The flow-through was captured. Then, the column was removed from magnet. 5 mL of PBS were poured onto the column and captured as eluate.
Between each run, the column was removed from the magnet and washed twice with water. All fractions were weighed in for possible yield calculation.

*Results and Discussion*

The results are depicted in Table 4 (ferritin in water) and Table 5 (cryo-poor plasma).

**Table 4.** Impact of magnetic forces on ferritin in water

| Sample | Iron conc. [$\mu$mol/L] | Volume [mL] | Total iron amount [$\mu$mol] | Yield [%] |
|---|---|---|---|---|
| Feed (ferritin in water) | 8.689 | 5.000 | 0.043 | - |
| Sample 1 flow-through | 6.486 | 4.960 | 0.032 | 74 |
| Sample 1 eluate | 1.415 | 5.080 | 0.007 | 17 |
| Sample 2 flow-through | 4.197 | 10.110 | 0.042 | 98 |
| Sample 2 eluate | 0.165 | 5.340 | 0.001 | 2 |

**Table 5.** Impact of magnetic forces on ferritin in cryo-poor plasma

| Sample | Iron conc. [$\mu$mol/L] | Volume [mL] | Total iron amount [$\mu$mol] | Yield [%] |
|---|---|---|---|---|
| Feed (ferritin in cryo-poor plasma) | 18.818 | 5.000 | 0.094 | - |
| Sample 1 flow-through | 20.321 | 4.830 | 0.098 | 104 |
| Sample 1 eluate | 2.186 | 5.400 | 0.012 | 13 |
| Sample 2 flow-through | 11.778 | 9.880 | 0.116 | 124 |
| Sample 2 eluate | 0.219 | 5.520 | 0.001 | 1 |

The magnetic column was not found to be suitible to capture ferritin at the given natural state of magnetic field strength. Only a slight retention of ferritin was found.

**Example 6**

**Proteins in PCC-depleted (prothrombin complex concentrate-depleated) cryo-poor plasma**

*Methods*

The device setup was as laid out above. PCC-depleted cryo-poor plasma and PCC-depleted cryo-poor plasma supplemented with ferritin was diluted 1:2 in PBS. 500 mL of each sample are pipetted into in a centrifugal column (300K NanoSep Filter) and were centrifuged for 20 min at 13,300 rpm. The small residue on the top of the filter was taken up again with 500 µL PBS Buffer and measured as well. Thus, two samples of filtrate and retentate with almost the same volumes were obtained. The samples are measured for total protein contents of immunoglobulins IgG, IgA, and IgM, albumin, ferritin and the total iron content.

*Results and Discussion*

The results are depicted in Table 6 (PCC-depleted cryo-poor plasma) and Table 7 (PCC-depleted cryo-poor plasma supplemented with ferritin).

**Table 6.** Protein contents in PCC-depleted cryo-poor plasma

|  | Feed | Filtrate | Retentate | Yield of Filtrate [%] | Yield of Retentate [%] |
|---|---|---|---|---|---|
| Total protein [g/L] | 30.655 | 8.831 | 19.427 | 29 | 63 |
| IgG [g/L] | 4.62 | 1.41 | 2.93 | 31 | 63 |
| IgA [g/L] | 0.881 | 0.272 | 0.537 | 31 | 61 |
| IgM [g/L] | 0.43 | 0.104 | 0.221 | 24 | 51 |
| albumin [g/L] | 17.411 | 5.224 | 10.912 | 30 | 63 |
| Ferritin [µg/L] | 16.433 | 4.846 | 10.413 | 29 | 63 |
| Iron content [µmol/L] | 7.255 | 2.78 | 4.975 | 38 | 69 |

**Table 7.** Protein contents in PCC-depleted cryo-poor plasma supplemented with ferritin

|  | Feed | Filtrate | Retentate | Yield of Filtrate [%] | Yield of Retentate [%] |
|---|---|---|---|---|---|
| Total protein [g/L] | 31.772 | 12.47 | 15.636 | 39 | 49 |
| IgG [g/L] | 4.66 | 1.7 | 2.34 | 36 | 50 |
| IgA [g/L] | 0.892 | 0.372 | 0.421 | 42 | 47 |
| IgM [g/L] | 0.378 | 0.159 | 0.206 | 42 | 54 |
| albumin [g/L] | 18.096 | 7.522 | 9.238 | 42 | 51 |
| Ferritin [µg/L] | 16.889 | 7.14 | 8.928 | 42 | 53 |
| Iron content [µmol/L] | 13.211 | 5.857 | 7.067 | 44 | 53 |

Since both fractions had the approximate same volume, the concentrations of these can be compared directly. The yields for the proteins in both fractions, filtrate and retentate, were in the same range for each of the samples. The yields of proteins in the respective filtrate or retentate did not correlate with the molecular weight of the proteins and their expected separation by the molecular weight cutoff. Ferritin with a molecular weight of more than 450 kDA was retained in a comparable percentage as albumin with a considerably lower molecular weight of 66 kDa.

**Example 7**

**Use reducing agents for removal of iron ions from ferritin by zeolites**

**Example 7A**

**Impact of ascorbic acid and citrate on iron ion removal by zeolites**

The device setup was as laid out above. Usability of ascorbate and ascorbate/citrate mixtures, respectively, was tested for its capability to mobilize iron from ferritin by reducing it.

*Methods*

Ferritin was added to solutions with different contents of ascorbic acid (5 mM), zeolites (UOP MOLSIV® TE 143 R) and optionally citrate (10 mM). The samples (50 mL in Falcon tubes) were put on a shaker and incubated at 4°C for 24 h. The remaining content of iron in the solution was measured afterwards.

*Results and Discussion*

The sample containing ferritin had an iron content of 0.694 $\mu$mol/L. The sample containing ferritin, 5 mM ascorbic acid and zeolites contained only 0.272 $\mu$mol/L of iron. The sample containing ferritin, 5 mM ascorbic acid, 10 mM citrate and zeolites contained 3.500 $\mu$mol/L of iron.

The iron concentration dropped significantly when adding 5 mM ascorbic acid and zeolites. The highest iron concentration was found when adding 5 mM ascorbic acid, 10 mM citrate and zeolites. The addition of ascorbic acid seems to help the release of incorporated iron ions from ferritin. Citrate chelates iron ions and therefore disturbs the withdrawal of iron ions by means of zeolites.

**Example 7B**

**Impact of different combinations of reducing agents on iron ion removal by zeolites**

*Method*

In order to investigate the impact of different combinations of reducing agents on iron ion removal by zeolites, a number of reducing agents and combinations thereof were investigated. The experimental setup is as laid out above. 10 $\mu$L of ferritin solution (10 $\mu$g/mL) were added to 50 mL of each sample. The Falcon tubes were closed, put on a shaker and slightly shaken for 24 hours at 4°C.

*Results and Discussion*

The results are depicted in Table 7.

**Table 7.** Different combinations of reducing agents and iron ion removal by zeolites (UOP MOLSIV® TE 143 R)

| Sample | 1 | 2 | 3 | 4 | 5 | 6 | 7 |
|---|---|---|---|---|---|---|---|
| 1 M urea | - | - | + | - | + | + | + |
| 5 mM ascorbic acid | - | - | - | + | + | - | + |
| 5 mM NADH/ 50 $\mu$M FMN | - | - | - | - | - | + | + |
| Zeolites | - | + | + | + | + | + | + |
| Total iron conc. [$\mu$g/L] | 0.770 | 1.194 | 0.940 | 0.134 | 0.232 | 1.636 | 0.680 |

Several iron concentrations were above the untreated feed reference sample 1. The samples containing ascorbic acid showed the lowest values iron values. Sample 6 containing NADH/FMN only showed a high iron concentration, whereas the combination of NADH/FMN and ascorbic acid (sample 7) showed a reduced iron concentration.

It was shown that zeolite with or without the aid of urea was did not show high activity to decrease iron concentration and to withdraw iron ions from ferritin. Ascorbic acid was found a highly effective reducing agent. It was found to have higher effectivity than the NADH/FMN reducing agent.

**Example 8**

**Evaluation of the release of iron ions by means of chelating agents**

**Example 8A**

**Chelating the iron(II)-irons with 2,2-bipyridine**

The release of iron ions was measured via chelating the iron(II)-irons with 2,2-bipyridine.

*Methods*

The experimental setup was as laid out above. A 20 mM 2,2-byridine solution was mixed 1:1 with 2 mM $FeCl_2$. The absorption peak was measured. After it was determined, a serial dilution of ferritin was performed to determine the lower quantification and detection limit.

*Results and Discussion*

As first part of the experiment the absorption spectra of the Fe-2,2-bipyridin-complex and the FMN/NaDH, were measured. The Fe-2,2-bipyridin-complex showed a maximum absorption of 530 nm, whereas the NADH/FMN system showed an increasing absorbance below around 510 nm with a maximum around 450 nm.

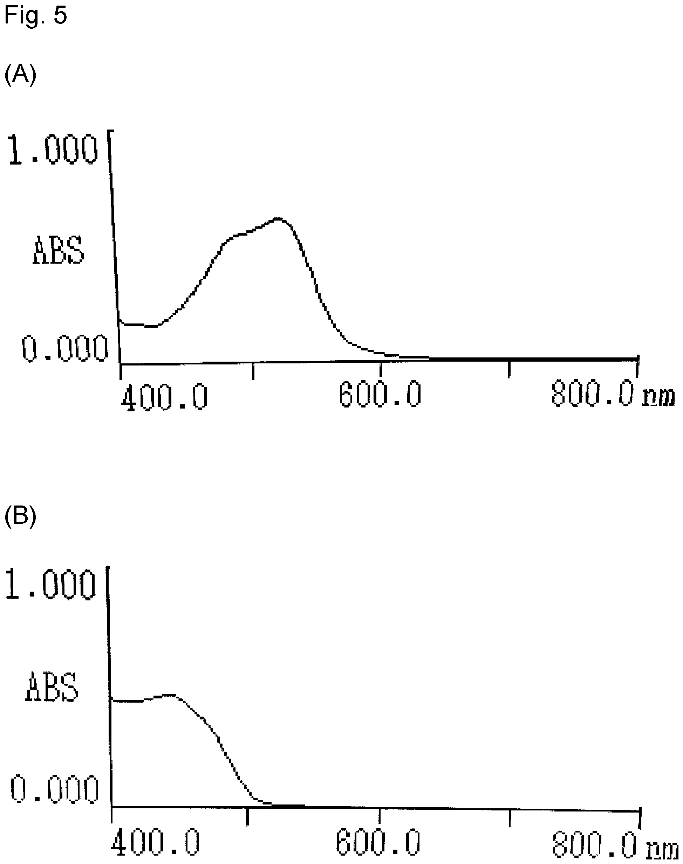

At 530 nm this NADH/FMN system showed essentially no absorbance. This is also depicted in Figure 5. Thus, essentially no unwanted interactions occurred when measuring the remaining iron ion concentration in the tested electron reduction systems (ascorbic acid and NADH/FMN).

Table 8. Measurement of Fe-2,2-bipyridin-complexes at 530 nm gave the following absorptions (2,2-bipyridin concentration was 10x above the iron ion concentration to assure that all available iron was complexed):

| Iron concentration | Absorption (530 nm) |
|---|---|
| 100 $\mu$mol/L | 1.025 |
| 10 $\mu$mol/L | 0.127 |
| 1 $\mu$mol/L | 0.006 (beyond detection limit) |
| 100 nmol/L | 0.003 (beyond detection limit) |
| 10 nmol/L | 0.032 (beyond detection limit) |

2,2-bipyridin was found to be usable as mean for detection of free iron ions in a solution. The usage of 2,2-bipyridin to measure iron ion concentrations via complex forming allows the measurement of free iron in solution, e.g., after being reduced and mobilized from ferritin. Since the NADH/FMN system seems to disturb the iron ion measurement, under certain measurement conditions, it may be beneficial to detect it by means of Fe-2,2-bipyridine complex formation. The extinction coefficient was calculated to be:

$$\varepsilon = \frac{E}{c \cdot d} = \frac{1.025}{0.0001 \frac{mol}{L} \cdot 1 cm} = 10250 \ \frac{L}{mol \cdot cm}$$

**Example 8B**

**Determining the capacity of reducing agents**

It was intended to determine the capability of reducing agents (also designatable as electron mediators in this context) to reduce the ferritin-incorporated iron ions and of zeolites to capture the released iron ions.

*Methods*

The device setup was as laid out above. 40 mL of four different samples as shown below were incubated for 3 hours after the addition of 40 $\mu$L ferritin solution (10 mg/mL) and their contents of free iron in the solution were measured. The four different samples were measured after the incubation by complexing the released iron with 2,2-bipyridine. The free iron ion concentrations of the samples that contain zeolites (UOP MOLSIV® TE 143 R) were below their respective counterparts without zeolites.

*Results and Discussion*

The samples were analyzed using spectrometry (absorption at 530 nm) to determine the Fe-2,2-bipyridin complex and the external iron assay of the clinical chemistry. The absorptions measured by the spectrometry at 530 nm were calculated into concentrations using the extinction coefficient, which was determined in Example 8A. The results are depicted in Table 9.

**Table 9.** Impact of reducing agents on zeolite-based withdrawal of iron ions

| Sample | Absorption (Fe-2,2-bipyridin complex) | Calculated iron conc. [$\mu$mol/L] | iron conc. (ext.) [$\mu$mol/L] |
|---|---|---|---|
| 5 mM ascorbic acid | 0.052 | 10.15 | 22.32 |
| 5 mM ascorbic acid and zeolite | 0.022 | 4.29 | 6.63 |
| 5 mM NADH and 50 $\mu$M FMN | -0.015 | - | 5.27 |
| 5 mM NADH, 50 $\mu$M FMN and zeolite | 0.017 | 3.32 | 5.36 |

Although the final concentrations of the calculations based on the Fe-2,2-bipyridine complex were below the results of the external measurement, these are still correlated in the relative sizes of the values.

It has been shown that ascorbic acid in combination with an iron-binding is highly effective for reducing iron ion content of ferritin. This mechanism was therefore proven for multiple times by the above experiments.

**Example 8C**

**Impact of pH, ferritin concentration and voltage on iron removal by means of zeolites**

The amount of added ferritin was increased, the pH reduced to pH 5, the voltage increased to force a stronger reduction of NADH and FMN and finally 2,2-bipyridin was used to measure the amount of released or remaining iron(II) ions in solution.

*Methods*

The device setup was as laid out above. 250 $\mu$L of an aqueous ferritin solution (10 $\mu$g/mL) were added to 50 mL of a solution with 2.5 mM NADH and 50 $\mu$M FMN. Samples were taken for analysis from the feed. The rest of the feed was splitted into two samples as depicted below. Afterwards the absorbances of both solutions were measured at 530 nm.

*Sample* 1: 0.2 g of 2,2-bypiridin was added to the ferritin solution, to be able to capture all of the released iron(II)-ions) in the solution. This demonstrated the capability of the NADH/FMN system to reduce the incorporated iron ions. The solution was then treated with a voltage of 3 V for 2 hours using the Voltcraft power supply.

*Sample 2:* Zeolite (UOP MOLSIV® TE 143 R) was added to the above solution and it was treated with a voltage of 3 V for 2 hours. After incubation, the zeolites were removed and the same amount of 2,2-bipyridin was added and the solution was mixed to chelate the reduced iron ions, which were not captured by the zeolites.

*Results and Discussion*

The feed ferritin solution (10 $\mu$g/mL) showed an absorption at 530 nm of 0.058. Sample 1 (addition of 2,2-bipyridine) showed an absorption at 530 nm of 0.122. Sample 2 (addition of 2,2-bipyridine and zeolite) showed an absorption at 530 nm of 0.363. Although Sample 1 showed a clearly visible red stain caused by the Fe-chelate-complex, the absorbance of Sample 2 was significantly higher. According to visual appearance of the solutions, it could be seen that sample 2 was more turbid causing the higher general absorbance of the sample. Both samples were filtered through a sterile filter of a pore size of 0.22 $\mu$m to remove any disturbances by zeolites or potential non-dissolved residues of the 2,2-bipyridine. The solutions were measured again afterwards.

After filtration, the untreated ferritin solution (10 $\mu$g/mL) showed an absorption at 530 nm of 0.062. Sample 1 (addition of 2,2-bipyridine) showed an absorption at 530 nm of 0.085. Sample 2 (addition of 2,2-bipyridine and zeolite) showed an absorption at 530 nm of 0.065. Therefore, after filtration, the absorbance of Sample 2 decreased significantly into range of the untreated ferritin solution. The absorbance of Sample 1 decreased, as well, but was as expected above the rest of samples. In addition, the iron contents of the solutions were determined using the external method of clinical chemistry of the Ferene® complex of the Siemens assay with the following results. In this assay, the untreated ferritin

solution (10 $\mu$g/mL) showed a concentration of 14.5 $\mu$mol/L. Sample 1 showed a concentration of 5.4 $\mu$mol/L. Sample 2 showed a concentration of 1.1 $\mu$mol/L. The resulting total concentrations of iron were reduced for both treated samples 1 and 2, also indicating that the iron ions that were already bound by the 2,2-bipyridine complex, were no longer available for binding with the Ferene® complex of the Siemens assay. The resulting iron concentration of Sample 2 with the zeolites and the later addition of 2,2-bipyridine was even below the concentration of Sample 1 with just the surplus of 2,2-bipyridine.

The visible reaction of the 2,2-bipyridine in Sample 1, proved the general ability of the NADH/FMN-system to work as reductive system for the generation of apoferritin. However, the low used voltage of former publications by Koochana et al. (Koochana et al.: Releasing iron from ferritin protein nanocage by reductive method: The role of electron transfer mediator, Biochemica et Biophysica Acta, 2018, 1862:1190-1198) did not lead to effective removal reduction and iron ion removal efficiency. It was found that voltage has to be significantly high enough to work against the oxidative effects by the dissolved oxygen and provide enough reducing effectivity of the NADH/FMN-system.

After removing the voltage to apply the reductive pressure on the reactants, reducing agents were still available in the solution to prevent a direct re-oxidation of the solved iron ions. Using the extinction coefficient from derived from Example 8A, the difference in the absorbance of 0.02 between Samples 1 (abs. 0.085) and Sample 2 (abs. 0.065), results in a concentration difference of

$$c = \frac{E}{\varepsilon \cdot d} = \frac{0{,}02}{10250 \; \frac{L}{mol \cdot cm} \cdot 1 \; cm} = 0.00000195 \; \frac{mol}{L} = 1.95 \; \frac{\mu mol}{L} \approx 2 \; \frac{\mu mol}{L}$$

The value of 2 $\mu$mol/L, which was derived from the low absorbances of the 2,2-bipyridine-complexes, was in the same range of the measured difference of 4.3 $\mu$mol/L between the two permutations by the assay of the Ferene® complex of the Siemens assay.

In summary, it was experimentally evidenced that a method based on an iron ion-adsorbing zeolite as claimed enables to effectively decreasing iron content of ferritin, in particular when a ferritin-containing fluid is used in a reducing environment containing one or more reducing agents (here exemplified as ascorbic acid).

## Claims

1. A *in vitro* method for decreasing iron content of ferritin, wherein said method comprises the following steps:

   (i) providing:

   (A) a ferritin-containing fluid containing one or more reducing agents suitable for reducing $Fe^{3+}$ ions to $Fe^{2+}$ ions,
   (B) a semipermeable layer, which is permeable for iron ions, but which is impermeable for polypeptides having a molecular weight of more than 300 kDa,
   (C) a zeolite suitable for adsorbing the iron ions which is suspended in water or an aqueous buffer, and

   (ii) placing the semipermeable layer between the ferritin-containing fluid and the zeolite so that the ferritin-containing fluid does not get in direct contact with the zeolite; and
   (iii) incubating the arrangement obtained from step (ii) under conditions that allow migration of the iron ions through the membrane and adsorption thereof to the zeolite.

2. The method of claim 1, wherein the method further comprises contacting a cathode with the ferritin-containing fluid and an anode with the water or aqueous buffer in which the zeolite is suspended and applying a voltage suitable for reducing $Fe^{3+}$ ions to $Fe^{2+}$ ions and/or suitable for reducing the one or more reducing agents.

3. The method of any one of claims 1 or 2, wherein the zeolite is also suitible for further adsorbing one or more further inorganic ions, in particular inorganic ions selected from the group consisting of zinc ions, calcium ions, aluminum ions, plutonium ions, cesium ions, beryllium ions, cupper ions, zinc ions, and calcium and zinc ions.

4. The method of any of claims 1 to 3, wherein the ferritin-containing fluid is a body fluid, preferably is a body fluid selected from the group consisting of blood, blood plasma or a fraction thereof, ascites, liquor cerebrospinalis, or the ferritin-containing fluid is selected from the group consisting of intracellular or extracellular liquids, cell culture fluids, homogenates or organs or tissues, hemolymph or a fraction thereof, and plant extracts, in particular wherein the ferritin-containing fluid is a unit of stored blood or plasma.

5. The method of any of claims 1 to 4, wherein the zeolite is **characterized by** at least one of the following: it is provided as a powder, in particulate form or as a paste; it has a pore size in the range of 140 to 600 pm, in particular 300 to 500 pm; and/or it is suspended in an aqueous buffer.

6. The method of any of claims 1 to 5, wherein the semipermeable layer has a cutoff for polypeptides in the range of 1 to 300 kDa, 2 to 250 kDa, 2 to 200 kDa, 2 to 150 kDa, 2 to 100 kDa, 2 to 50 kDa, 2 to 45 kDa, 20 to 40 kDa, 25 to 35 kDa, or 5 to 15 kDa and/or where the semipermeable layer is an osmosis membrane.

7. The method of any of claims 1 to 6, wherein the semipermeable layer forms part of a device selected from the group consisting of a filter, in particular a filter selected from the group consisting of a centrifugation filter, a filter for dead-end filtration under standard pressure, high pressure or vacuum, and a cross-flow filtration filter; a dialysis device, in particular a dialysis device selected from the group consisting of a dialysis membrane, a dialysis tube, and a dialysis bag; and a hollow fibre.

8. The method of any of claims 1 to 7, wherein the method is **characterized by** at least one of the following: it is conducted in an on-line procedure; the ferritin-containing fluid flows alongside the semipermeable layer; the ferritin-containing fluid is subjected to apheresis; and/or the method is conducted on-line within an apheresis or blood donation procedure.

9. The method of any of claims 1 to 8, wherein the ferritin-containing fluid further comprises one or more chaotropic agents decreasing interactions between ferritin sub-units and/or releasing iron ions from ferritin.

10. The method of any of claims 1 to 9, wherein the step (iii) of incubating is conducted:

for at least 5 min, at least 10 min, or at least 20 min; and/or until the concentration of iron ions in the ferritin-containing fluid is reduced by at least 25% (mol/mol) in comparison to the concentration contained in the ferritin-containing fluid before conducting said method.

11. The method of any of claims 1 to 10, wherein the method further comprises the addition of one or more complexing agents which complex one or more species of ions.

12. An in vitro method for reducing the concentration of iron ions in a body fluid, after intoxication, wherein said method is conducted in accordance with any one of claims 1 to 11, preferably wherein said method further comprises using a dialysis procedure, in particular a peritoneal dialysis procedure for detoxification.

**Patentansprüche**

1. In-vitro-Verfahren zur Verringerung des Eisengehalts von Ferritin, wobei das Verfahren die folgenden Schritte umfasst:

(i) Bereitstellen:

(A) eines ferritinhaltigen Fluides, das ein oder mehrere Reduktionsmittel enthält, die geeignet sind, $Fe^{3+}$-Ionen zu $Fe^{2+}$-Ionen zu reduzieren,
(B) einer semipermeablen Schicht, die für Eisenionen durchlässig ist, die aber für Polypeptide mit einem Molekulargewicht von mehr als 300 kDa undurchlässig ist,
(C) eines zur Adsorption der Eisenionen geeigneten Zeolithen, der in Wasser oder einem wässrigen Puffer suspendiert ist, und

(ii) Anordnen der semipermeablen Schicht zwischen dem ferritinhaltigen Fluid und dem Zeolithen, so dass das ferritinhaltige Fluid nicht in direkten Kontakt mit dem Zeolithen kommt; und
(iii) Inkubieren der aus Schritt (ii) erhaltenen Anordnung unter Bedingungen, die eine Migration der Eisenionen durch die Membran und deren Adsorption an den Zeolithen erlauben.

2. Verfahren nach Anspruch 1, wobei das Verfahren ferner das Inkontaktbringen einer Kathode mit dem ferritinhaltigen Fluid und einer Anode mit dem Wasser oder wässrigen Puffer, in dem der Zeolith suspendiert ist, und das Anlegen einer Spannung, die geeignet ist, $Fe^{3+}$-Ionen zu $Fe^{2+}$-Ionen zu reduzieren und/oder die geeignet ist, ein oder mehrere Reduktionsmittel zu reduzieren, umfasst.

3. Verfahren nach einem der Ansprüche 1 oder 2, wobei der Zeolith auch geeignet ist, ferner ein oder mehrere weitere anorganische Ionen zu adsorbieren, insbesondere anorganische Ionen, ausgewählt aus der Gruppe bestehend aus Zinkionen, Calciumionen, Aluminiumionen, Plutoniumionen, Cäsiumionen, Berylliumionen, Kupferionen, Zinkionen und Calcium- und Zinkionen.

4. Verfahren nach einem der Ansprüche 1 bis 3, wobei das ferritinhaltige Fluid eine Körperflüssigkeit ist, vorzugsweise eine Körperflüssigkeit, ausgewählt aus der Gruppe bestehend aus Blut, Blutplasma oder einer Fraktion davon, Aszites, Liquor cerebrospinalis, oder das ferritinhaltige Fluid ausgewählt ist aus der Gruppe bestehend aus intrazellulären oder extrazellulären Flüssigkeiten, Zellkulturflüssigkeiten, Homogenaten von Organen oder Geweben, Hämolymphe oder einer Fraktion davon, und Pflanzenextrakten, insbesondere wobei das ferritinhaltige Fluid eine Einheit von gelagertem Blut oder Plasma ist.

5. Verfahren nach einem der Ansprüche 1 bis 4, wobei der Zeolith **gekennzeichnet ist durch** mindestens eines der folgenden: er wird als Pulver, in Partikelform oder als Paste bereitgestellt; er hat eine Porengröße im Bereich von 140 bis 600 pm, insbesondere 300 bis 500 pm; und/oder er ist in einem wässrigen Puffer suspendiert.

6. Verfahren nach einem der Ansprüche 1 bis 5, wobei die semipermeable Schicht einen Ausschluss für Polypeptide im Bereich von 1 bis 300 kDa, 2 bis 250 kDa, 2 bis 200 kDa, 2 bis 150 kDa, 2 bis 100 kDa, 2 bis 50 kDa, 2 bis 45 kDa, 20 bis 40 kDa, 25 bis 35 kDa oder 5 bis 15 kDa aufweist und/oder wobei die semipermeable Schicht eine Osmosemembran ist.

7. Verfahren nach einem der Ansprüche 1 bis 6, wobei die semipermeable Schicht Teil einer Vorrichtung bildet, ausgewählt aus der Gruppe bestehend aus einem Filter, insbesondere einem Filter ausgewählt aus der Gruppe bestehend aus einem Zentrifugalfilter, einem Filter für Kuchenfiltration unter Standarddruck, Hochdruck oder Vakuum, und einem Querstromfiltrationsfilter; einer Dialysevorrichtung, insbesondere einer Dialysevorrichtung ausgewählt aus der Gruppe bestehend aus einer Dialysemembran, einem Dialyseschlauch und einem Dialysebeutel; und einer Hohlfaser.

8. Verfahren nach einem der Ansprüche 1 bis 7, wobei das Verfahren **gekennzeichnet ist durch** mindestens eines der folgenden: es wird in einem On-Line-Prozess durchgeführt; das ferritinhaltige Fluid fließt entlang der semipermeablen Schicht; das ferritinhaltige Fluid wird einer Apherese unterzogen; und/oder das Verfahren wird on-line innerhalb eines Apherese- oder Blutspendeprozesses durchgeführt.

9. Verfahren nach einem der Ansprüche 1 bis 8, wobei das ferritinhaltige Fluid ferner ein oder mehrere chaotrope Mittel enthält, die die Wechselwirkungen zwischen Ferritin-Untereinheiten verringern und/oder Eisenionen aus Ferritin freisetzen.

10. Verfahren nach einem der Ansprüche 1 bis 9, wobei der Schritt (iii) des Inkubierens durchgeführt wird:

   für mindestens 5 min, mindestens 10 min oder mindestens 20 min; und/oder
   bis die Konzentration der Eisenionen in dem ferritinhaltigen Fluid um mindestens 25 % (mol/mol) im Vergleich zu der Konzentration, die in dem ferritinhaltigen Fluid vor Durchführen des Verfahrens enthalten war, reduziert ist.

11. Verfahren nach einem der Ansprüche 1 bis 10, wobei das Verfahren ferner die Zugabe von einem oder mehreren Komplexbildnern, die eine oder mehrere Arten von Ionen komplexieren, umfasst.

12. *In-vitro*-Verfahren zur Verringerung der Konzentration von Eisenionen in einer Körperflüssigkeit nach einer Intoxikation, wobei das Verfahren entsprechend einem der Ansprüche 1 bis 11 durchgeführt wird, bevorzugt wobei das Verfahren ferner die Verwendung eines Dialyseprozesses, insbesondere eines Peritonealdialyseprozesses zur Entgiftung, umfasst.

**Revendications**

1. Procédé *in vitro* de réduction de la teneur en fer de la ferritine, ledit procédé comprenant les étapes suivantes :

(i) la fourniture de :

(A) un fluide contenant de la ferritine contenant un ou plusieurs agents réducteurs permettant de réduire les ions $Fe^{3+}$ en ions $Fe^{2+}$,
(B) une couche semi-perméable, qui est perméable aux ions fer, mais imperméable aux polypeptides de poids moléculaire supérieur à 300 kDa,
(C) une zéolite adsorbant les ions fer, qui est en suspension dans l'eau ou un tampon aqueux, et

(ii) le placement de la couche semi-perméable entre le fluide contenant de la ferritine et la zéolite, de sorte que le fluide contenant de la ferritine n'entre pas en contact direct avec la zéolite ; et
(iii) l'incubation de l'agencement obtenu à l'étape (ii) dans des conditions qui permettant la migration des ions fer à travers la membrane et leur adsorption sur la zéolite.

2. Procédé selon la revendication 1, le procédé comprenant en outre la mise en contact d'une cathode avec le fluide contenant de la ferritine et d'une anode avec l'eau ou le tampon aqueux dans lequel la zéolite est en suspension, et l'application d'une tension appropriée pour réduire les ions $Fe^{3+}$ en ions $Fe^{2+}$ et/ou appropriée pour réduire les un ou plusieurs agents réducteurs.

3. Procédé selon l'une quelconque des revendications 1 ou 2, dans lequel la zéolite est également adaptée pour l'adsorption d'un ou plusieurs ions inorganiques supplémentaires, notamment des ions inorganiques choisis dans le groupe constitué par les ions zinc, les ions calcium, les ions aluminium, les ions plutonium, les ions césium, les ions béryllium, les ions cuivre, les ions zinc et les ions calcium et zinc.

4. Procédé selon l'une quelconque des revendications 1 à 3, dans lequel le fluide contenant de la ferritine est un fluide corporel, de préférence un fluide corporel choisi dans le groupe constitué par le sang, le plasma sanguin ou une fraction de celui-ci, les ascites, le liquide céphalorachidien, ou le fluide contenant de la ferritine est choisi dans le groupe constitué par des liquides intracellulaires ou extracellulaires, des fluides de culture de cellules, des homogénats d'organes ou de tissus, l'hémolymphe ou une fraction de celle-ci, et des extraits de plantes, le fluide contenant de la ferritine étant en particulier une unité de sang ou de plasma stocké.

5. Procédé selon l'une quelconque des revendications 1 à 4, dans lequel la zéolite est **caractérisée par** au moins l'un des suivants : elle est fournie sous forme de poudre, sous forme de particules ou sous forme de pâte ; elle a une taille de pores dans la plage de 140 à 600 pm, notamment de 300 à 500 pm ; et/ou elle est mise en suspension dans un tampon aqueux.

6. Procédé selon l'une quelconque des revendications 1 à 5, dans lequel la couche semi-perméable présente un seuil de coupure pour les polypeptides dans la plage de 1 à 300 kDa, 2 à 250 kDa, 2 à 200 kDa, 2 à 150 kDa, 2 à 100 kDa, 2 à 50 kDa, 2 à 45 kDa, 20 à 40 kDa, 25 à 35 kDa ou 5 à 15 kDa, et/ou dans lequel la couche semi-perméable est une membrane d'osmose.

7. Procédé selon l'une quelconque des revendications 1 à 6, dans lequel la couche semi-perméable fait partie d'un dispositif choisi dans le groupe constitué par un filtre, en particulier un filtre choisi dans le groupe constitué par un filtre de centrifugation, un filtre de filtration frontale à pression standard, à haute pression ou sous vide, et un filtre de filtration tangentielle ; un dispositif de dialyse, notamment un dispositif de dialyse choisi parmi une membrane de dialyse, un tube de dialyse et une poche de dialyse ; et une fibre creuse.

8. Procédé selon l'une quelconque des revendications 1 à 7, le procédé étant **caractérisé par** au moins l'un des suivants : il est conduit dans une procédure en ligne ; le fluide contenant de la ferritine s'écoule le long de la couche semi-perméable ; le fluide contenant de la ferritine est soumis à une aphérèse ; et/ou le procédé est conduit en ligne dans le cadre d'une procédure d'aphérèse ou de don de sang.

9. Procédé selon l'une quelconque des revendications 1 à 8, dans lequel le fluide contenant de la ferritine comprend en outre un ou plusieurs agents chaotropiques diminuant les interactions entre les sous-unités de la ferritine et/ou libérant des ions fer à partir de la ferritine.

10. Procédé selon l'une quelconque des revendications 1 à 9, dans lequel l'étape (iii) d'incubation est conduite :

pendant au moins 5 min, au moins 10 min ou au moins 20 min ; et/ou

jusqu'à ce que la concentration d'ions fer dans le fluide contenant de la ferritine soit réduite d'au moins 25 % (mol/mol) par rapport à la concentration contenue dans le fluide contenant de la ferritine avant la conduite dudit procédé.

11. Procédé selon l'une quelconque des revendications 1 à 10, le procédé comprenant en outre l'ajout d'un ou plusieurs agents complexants qui complexent une ou plusieurs espèces d'ions.

12. Procédé in vitro pour réduire la concentration d'ions fer dans un fluide corporel, après une intoxication, ledit procédé étant conduit selon l'une quelconque des revendications 1 à 11, ledit procédé comprenant de préférence en outre l'utilisation d'une procédure de dialyse, en particulier une procédure de dialyse péritonéale pour la détoxification.

Fig. 1

Fig. 2

Fig. 3

Fig. 4

Fig. 5

(A)

(B)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5358722 A

- JP 2004217984 A

### Non-patent literature cited in the description

- **WANG et al.** Serum ferritin: Past, present and future. *Biochimica et Biophysica Acta*, vol. 1800 (8), 760-769
- **THEIL**. Ferritin protein nanocages - the story. *Nanotechnology Perceptions*, 2012, vol. 8 (1), 7-16
- **HARRISON PM** ; **AROSIO P**. The ferritins: molecular properties, iron storage function and cellular regulation. *Biochim. Biophys. Acta*, 1996, vol. 1275, 161-203
- **REGAN RF et al.** Ferritin induction protects cortical astrocytes from heme-mediated oxidative injury. *Neuroscience*, 2002, vol. 113 (4), 985
- **SHACKELFORD RE et al.** Pharmacological manipulation of ataxia-telangiectasia kinase activity as a treatment for Parkinson's disease. *Medical Hypotheses*, 2005, vol. 64, 736-741
- **FRIEDMAN A et al.** Iron as a cause of Parkinson's disease - a myth or a well-established hypothesis?. *Parkinsonism Relat Disord.*, December 2009, vol. 15 (3), 212-4
- **MOSTILE G**. Iron and Parkinson's disease: A systematic review and meta-analysis. *Mol Med Rep.*, May 2017, vol. 15 (5), 3383-3389

- **GARRINGER et al.** Effect of Systemic Iron Overload and a Chelation Therapy in a Mouse Model of the Neurodegenerative Disease Hereditary Ferritinopathy. *PlosOne*, 2016, vol. 11 (8), e0161341
- **MOGLIA et al.** *Journal of Inorganic Biochemistry*, 2018, vol. 188, 184-190
- **PARVU et al.** Ferritin level changes and erythroid improvement in a group of adult polytransfused patients treated with Deferasirox. *Clujul Med.*, July 2018, vol. 91 (3), 288-292
- **ALAM et al.** Hemorrhage Control in the Battlefield: Role of New Hemostatic Agents. *Military Medicine*, 2005, vol. 170, 63-69
- **LI et al.** Zeolite-based hemostat QuikClot releases calcium into blood and promotes blood coagulation in vitro. *Acta Pharmacologica Sinica*, 2013, vol. 34, 367-372
- **KOOCHANA et al.** Releasing iron from ferritin protein nanocage by reductive method: The role of electron transfer mediator. *Biochemica et Biophysica Acta*, 2018, vol. 1862, 1190-1198